(19) Europäisches Patentamt
European Patent Office
Office européen des brevets

(11) **EP 4 191 593 A1**

(12) **EUROPEAN PATENT APPLICATION**
published in accordance with Art. 153(4) EPC

(43) Date of publication:
**07.06.2023 Bulletin 2023/23**

(21) Application number: **21854354.4**

(22) Date of filing: **30.07.2021**

(51) International Patent Classification (IPC):
***G16B 5/00*** *(2019.01)* ***A61B 5/00*** *(2006.01)*
***A61P 23/00*** *(2006.01)*

(52) Cooperative Patent Classification (CPC):
**A61B 5/00; A61P 23/00; G16B 5/00**

(86) International application number:
**PCT/CN2021/109601**

(87) International publication number:
**WO 2022/028322 (10.02.2022 Gazette 2022/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.08.2020 CN 202010766611**

(71) Applicant: **Sichuan Haisco Pharmaceutical Co., Ltd.**
**Sichuan 611130 (CN)**

(72) Inventors:
- **WANG, Xu**
  **Chengdu City, Sichuan 611130 (CN)**
- **JI, Qianqian**
  **Chengdu City, Sichuan 611130 (CN)**
- **CHEN, Kai**
  **Chengdu City, Sichuan 611130 (CN)**
- **LIU, Xiao**
  **Chengdu City, Sichuan 611130 (CN)**
- **YAN, Pangke**
  **Chengdu City, Sichuan 611130 (CN)**
- **WU, Nan**
  **Chengdu City, Sichuan 611130 (CN)**

(74) Representative: **Hoffmann Eitle**
**Patent- und Rechtsanwälte PartmbB**
**Arabellastraße 30**
**81925 München (DE)**

# (54) METHOD AND SYSTEM FOR DETERMINING POPULATION PHARMACOKINETIC MODEL OF PROPOFOL AND DERIVATIVE THEREOF

(57) Provided are a method and system for determining a population pharmacokinetic model of propofol and a derivative thereof. The method comprises determining a population pharmacokinetic model of a compound of formula (I) or propofol, wherein an equation of pharmacokinetic parameters in the population pharmacokinetic model of the compound of formula (1) comprises:

$$CL_i = \exp(4.20{+}0.349 \cdot \log(WT / 63.9) - 0.749 \cdot \log(TP / 72.4) + 0.238 \cdot SITE + \eta_{CL,i})\ ;$$

an equation of pharmacokinetic parameters in the population pharmacokinetic model of propofol comprises:

$$CL_i = \exp(4.56 + \eta_{CL,i})\ .$$

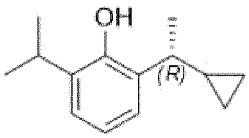

(I).



EP 4 191 593 A1

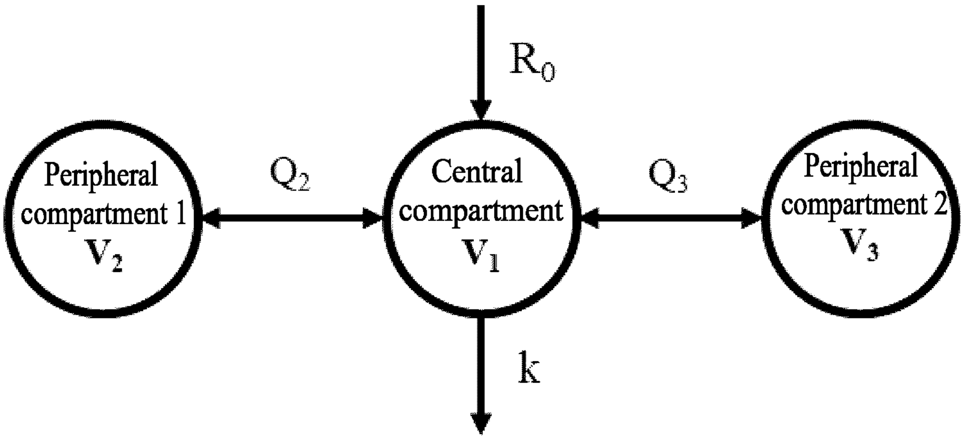
R0
Q2
Q3
Peripheral compartment 1 V2
Central compartment V1
Peripheral compartment 2 V3
k

*FIG. 1*

## Description

### Technical Field

The present invention relates to the field of medicine, and specifically relates to a method and system for determining a population pharmacokinetic model of propofol and a derivative thereof.

### Background Art

Propofol derivative injectable emulsion (hereinafter referred to as propofol derivative, with a chemical name of 2-[(1R)-1-cyclopropylethyl]-6-isopropyl-phenol), which is developed by Sichuan Haisco Pharmaceutical Co., Ltd. as a novel intravenous anesthetic with independent intellectual property rights, is intended to be used for sedation/anesthesia in various diagnostic examinations or treatments, induction and maintenance of general anesthesia, and sedation (ICU sedation) of intensive care subjects under mechanical ventilation. The propofol derivative, as the active ingredient, is a chemical entity similar to propofol, and is a single diastereomer with two chiral centers of R-configuration. The strategy of the medicinal chemistry design is to systematically improve the pharmacological and physicochemical properties of the drug for binding to a receptor to obtain a compound superior to propofol, i.e., the propofol derivative. The main action mechanism of the propofol derivative is to allow for the inflow of chloride ions by enhancing an ion channel mediated by a $\gamma$-aminobutyric acid type A (GABAA) receptor, thereby achieving the inhibition of the nervous centralis. The channel is also a main target where propofol exerts its effects. The propofol derivative has the pharmacodynamic characteristics of fast onset of action and stable and rapid recovery. Moreover, the propofol derivative has higher target selectivity and *in-vitro* and *in-vivo* activities and a potency 4-5 times that of propofol, and shows a more stable hemodynamics in animal trials. In addition, under the same conditions and concentrations, it is measured by using "an ultrafiltration method" that the propofol derivative has a lower free drug concentration in the aqueous phase as compared with propofol (Jingan®), suggesting that pain at an injection site may be reduced or eliminated.

### Summary of the Invention

An objective of the present invention is to provide a method for determining a population pharmacokinetic model of a propofol derivative.

An objective of the present invention is to quantitatively evaluate the influence of both intrinsic and extrinsic factors on the PK by using the method of the present invention. According to the population pharmacokinetic (PopPK) model established in the present invention, the individual exposure can be estimated for exposure-response (E-R) analysis. E-R analysis is critical for understanding the drug safety and efficacy. Dosage is a direct indicator of drug exposure commonly used in clinical trials, but the drug concentration in serum/plasma is a more direct indicator of exposure to a target of drug action and thus is correlated with the clinical efficacy and safety.

Another objective of the present invention is to provide a system for determining clinical individual administration parameters of a compound of formula (I) or propofol.

In order to achieve the above-mentioned objectives, on one hand, the present invention provides a method for determining a population pharmacokinetic model of a compound of formula (I) (the propofol derivative). The method comprises determining the population pharmacokinetic model of the compound of formula (I):

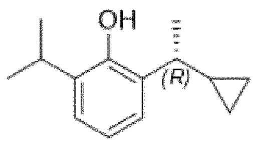
formula (I)

wherein an equation of pharmacokinetic parameters in the population pharmacokinetic model of the compound of formula (1) comprises:

$$CL_i = \exp(4.20 + 0.349 \cdot \log(WT / 63.9) - 0.749 \cdot \log(TP / 72.4) + 0.238 \cdot SITE + \eta_{CL,i})$$
;

wherein CLi represents central compartment clearance of the ith subject; when collecting a sample from venous blood, SITE = 0, and when collecting a sample from artery blood, SITE = 1; WT represents weight; TP represents total protein;

$\eta_{CL,i}$ is interindividual variation of the CL of the ith subject; and η follows a normal distribution with mean 0 and variance $\omega_2$, wherein the ω2 is a diagonal element of a variance-covariance matrix (S2) of the interindividual variation.

The present invention provides a method for determining a population pharmacokinetic model of propofol. The method comprises determining the population pharmacokinetic model of propofol, wherein an equation of pharmacokinetic parameters in the population pharmacokinetic model of propofol comprises:

$$CL_i = \exp(4.56 + \eta_{CL,i})$$

wherein CLi represents central compartment clearance of the ith subject; $\eta_{CL,i}$ is interindividual variation of the CL of the ith subject; and η follows a normal distribution with mean 0 and variance ω2, wherein the ω2 is a diagonal element of a variance-covariance matrix (S2) of the interindividual variation.

The above-mentioned model shows the relationship between the drug clearance and related covariates (weight, total protein and administration site). The influence of the covariates on the PK parameters (mainly the drug exposure) can be clinically evaluated by the model so as to guide the clinical administration. Firstly, the individual PK parameters of a subject are estimated by using a Bayesian post-hoc method on the basis of the PopPK model established in this study, and a plasma drug concentration-time curve of the propofol derivative or propofol administered by intravenous infusion is simulated according to the actual dosages of administration so as to calculate the area (the exposure) under the plasma drug concentration-time curve in different time periods. In addition, it is necessary to combine with the data analysis of correlation between the exposure and the drug efficacy and safety to give a reasonable dosage regimen.

The above-mentioned population pharmacokinetic models (PopPK models) of the present invention are ultimately selected from a three-compartment model with zero-order absorption and first-order linear elimination from the central compartment (as shown in Figure 1). The PopPK model is composed of the following parameters: central compartment clearance (CL), volume of distribution in the central compartment (V1), volumes of distribution in the peripheral compartments (V2, V3), and intercompartmental clearances (Q2, Q3).

According to some specific embodiments of the present invention, the equation of the pharmacokinetic parameters in the population pharmacokinetic model of the compound of formula (1) further comprises:

$$V_{1i} = \exp(0.908 + 0.426 \cdot \log(AGE / 27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and η represents interindividual variation of a corresponding parameter.

According to some specific embodiments of the present invention, the equation of the pharmacokinetic parameters in the population pharmacokinetic model of propofol further comprises:

$$V_{1i} = \exp(2.25 + \eta_{V_1,i})$$

$$Q_{2i} = \exp(4.96)$$

$$V_{2i} = \exp(3.63)$$

$$Q_{3i} = \exp(3.88)$$

$$V_{3i} = \exp(5.57)$$

According to some specific embodiments of the present invention, the method for determining the population pharmacokinetic model of the compound of formula (I) and propofol comprises obtaining the population pharmacokinetic model according to the influence of the covariates on the pharmacokinetic parameters in the population pharmacokinetic model of the compound of formula (I) and propofol.

According to some specific embodiments of the present invention, the method for determining the population pharmacokinetic model of the compound of formula (I) and propofol comprises the following steps (the population pharmacokinetic model of the compound of formula (I) and propofol can be determined by using a method comprising the following steps):

(1) acquisition of data;
(2) determination of data included in an analysis;
(3) processing of data;
(4) establishment of a preliminary population pharmacokinetic foundation model;
(5) establishment of a final population pharmacokinetic foundation model;
(6) establishment of the population pharmacokinetic model; and
(7) evaluation of the population pharmacokinetic model.

It can be understood that the serial numbers (1), (2), (3), ... etc. before the above-mentioned steps of the present invention should be understood as the numbers of various steps, and should not be understood as a limitation on the order of the steps.

According to some specific embodiments of the present invention, the above-mentioned steps can be performed sequentially according to the order as described above.

According to some specific embodiments of the present invention, the data in step (1) are derived from clinical trial data.

According to some specific embodiments of the present invention, step (2) comprises determining a pharmacokinetic data set included in the analysis by evaluating the clinical trial data included.

According to some specific embodiments of the present invention, the data included in the analysis in step (2) comprise plasma drug concentration data, baseline demographic characteristic data, blood biochemical index data and blood collection sites.

According to some specific embodiments of the present invention, the baseline demographic characteristic data comprise any combination of two or more of race, age, height, weight and gender; and the blood biochemical index data comprise any combination of two or more of blood total protein content, creatinine clearance, glutamic-oxalacetic transaminase, glutamic-pyruvic transaminase, alkaline phosphatase and total bilirubin.

According to some specific embodiments of the present invention, step (2) comprises determining a pharmacokinetic data set included in the analysis by evaluating the clinical trial data included.

According to some specific embodiments of the present invention, step (3) comprises determining and processing one or a combination of two or more of observations below a lower limit of detection, anomalous value data, outliers and missing covariates.

According to some specific embodiments of the present invention,

determining and processing the observations below the lower limit of detection comprises: determining the lower limit of detection by a detecting instrument, wherein the observations below the lower limit of detection are not used for population pharmacokinetic analysis, and if the proportion of the observations below the lower limit of detection is greater than 15%, investigating the influence of the observations below the lower limit of detection on model goodness-of-fit and modeling parameters by using a likelihood function method;

determining and processing the anomalous value data comprises: checking whether there are anomalous values

in a sample according to a plasma concentration - time curve in subjects receiving drug administration, and eliminating the anomalous values;

determining and processing the outliers comprises: determining outliers according to residual analysis of preliminary modeling results, and eliminating the outliers; and

processing the missing covariates comprises: if the missing covariate for the subject is less than 15%, applying imputation with the median of the data set for continuous covariates, and applying imputation with a value of the most common category for categorical covariates; and if the missing covariate for the subject is greater than 15%, applying no imputation, and performing exploratory analysis on the PK parameters of the subject with full covariate information by using a Bayesian estimation method.

According to some specific embodiments of the present invention, the data lower than LLOQ in pharmacokinetic samples only account for 6.8% (167/2463), so a M3 method does not need to be used.

According to some specific embodiments of the present invention, the anomalous value data comprise:

1) repeated concentration records at the same time point, except for concentrations in the artery and the vein at the same time point;
2) a trough concentration greater than a corresponding peak concentration;
3) an administration time point after the peak concentration;
4) an administration time point before the trough concentration;
5) concentration records after the end of intravenous administration and before the peak concentration; and
6) unexplained sudden drop or sudden rise in concentrations.

According to some specific embodiments of the present invention, for a method for confirming details of outliers, reference is made to the standard PopPK guidelines (Guidance for Industry: Population Pharmacokinetics issued by the U.S. Food and Drug Administration (FDA) and Guideline on Reporting the Results of Population Pharmacokinetic Analysis issued by the Committee for Medicinal Products for Human Use (CHMP)).

According to some specific embodiments of the present invention, the outliers are data points outside the specification range of a data set and are determined according to the residual analysis of the preliminary modeling results.

According to some specific embodiments of the present invention, if an absolute value of a conditional weighted residual (CWRES) is greater than 5, the data point is regarded as an outlier and deleted from PopPK modeling. After the final population pharmacokinetic model is determined, the outliers are added to the analysis data for reconstruction of a model, so as to evaluate whether the outliers have an influence on the model.

According to some specific embodiments of the present invention, step (4) comprises comparing various structural models on the basis of a plasma drug concentration-time curve, wherein the optimal model is selected as a preliminary structural model, to form a preliminary foundation model with a residual model.

According to some specific embodiments of the present invention, the preliminary structural model is a three-compartment model (as shown in Figure 1) with zero-order absorption and first-order linear elimination from the central compartment, and parameters of the preliminary structural model comprise:

central compartment clearance, CL; volume of distribution in the central compartment, V1; volume of distribution in the peripheral compartment 1, V2; volume of distribution in the peripheral compartment 2, V3; intercompartmental clearance between the peripheral compartment 1 and the central compartment, Q2; intercompartmental clearance between the peripheral compartment 2 and the central compartment, Q3; infusion rate, R0; and elimination rate constant K.

According to some specific embodiments of the present invention, with the preliminary foundation model, interindividual variation of the PK parameters is described by using the following equation:

$$\theta_i = \exp(\theta_T + \eta_i)$$

wherein $\theta i$ represents a PK parameter of the ith subject; $\theta_T$ represents a natural logarithm of a population typical value of the PK parameter; and $\eta_i$ represents interindividual variation, and is a random variable following a normal distribution with mean 0 and variance $\omega^2$, wherein the $\omega^2$ value represents a diagonal element of a variance-covariance matrix of the interindividual variation.

According to some specific embodiments of the present invention, with the preliminary foundation model, variability of a residual is described by using the following equation:

$$log\left(y_{ij}\right) = log\left(\hat{y}_{ij}\right) + \varepsilon_{ij}$$

wherein $y_{ij}$ represents the jth observed concentration of the ith subject, $\hat{y}_{ij}$ represents the jth model-predicted concentration of the ith subject, and $\varepsilon_{ij}$ represents a proportional residual of the jth observed concentration of the ith subject, wherein the observed concentration and the predicted concentration are independent of each other and follow a normal distribution with mean 0 and variance σ2, respectively.

According to some specific embodiments of the present invention, step (4) comprises comparing any two or more selected from the structural models of a one-compartment model, a two-compartment model and a three-compartment model on the basis of the plasma drug concentration-time curve, and selecting the best preliminary structural model.

According to some specific embodiments of the present invention, step (4) comprises comparing various structural models by comparing whether the decrease of objective function values of two nested models is significant, and selecting the best preliminary structural model.

According to some specific embodiments of the present invention, step (5) comprises determining covariates included in the evaluation on the basis of clinical knowledge and a drug action mechanism, and establishing a final population pharmacokinetic foundation model on the basis of the covariates included in the evaluation.

According to some specific embodiments of the present invention, the covariates included in the evaluation comprise baseline demographic characteristic covariates, blood biochemical index covariates and blood collection sites.

According to some specific embodiments of the present invention, the baseline demographic characteristic covariates comprise any combination of two or more of baseline values for age, gender, weight and race; and the blood biochemical index covariates comprise any combination of two or more of creatinine clearance, total protein, glutamic-oxalacetic transaminase, glutamic-pyruvic transaminase, alkaline phosphatase and total bilirubin.

According to some specific embodiments of the present invention, step (6) comprises:

a) pre-screening of covariates; and
b) final screening of the covariates by using a forward method and a backward method, and establishing the population pharmacokinetic model.

According to some specific embodiments of the present invention, the pre-screening of the covariates comprises: analyzing the correlation between the PK parameters and each covariate by a graphic method, using linear regression for continuous covariates and using an analysis of variance test for categorical covariates; evaluating the data set on the basis of the model; estimating the parameters of the subject with the final foundation model by using a Bayesian estimation method; and estimating the influence of the covariates on the PK parameters.

According to some specific embodiments of the present invention, the pre-screening of the covariates comprises:

analyzing the correlation between the continuous covariates and the PK parameters by using the following equation:

$$\theta_i = \theta_{pop} \cdot \left( \frac{Cov_i}{Cov_{pop}} \right)^{k_{\mathrm{cov}}};$$

and analyzing the correlation between the categorical covariates and the PK parameters by using the following equation:

$$\theta_i = \theta_{pop} \times \exp(k_{\mathrm{cov}} \cdot X_i)$$

wherein $\theta_i$ represents a PK parameter of the ith subject; $\theta_{pop}$ represents a test population typical value of the PK parameter; $Cov_i$ represents a continuous covariate of the ith subject; $Cov_{pop}$ represents a median of the continuous variables in a test population; $X_i$ represents a categorical variable index of the ith subject, wherein value 0 represents the category with the most common category of a covariate, while other integer values represent other categories respectively; and $K_{cov}$ represents a coefficient describing the influence of the covariate.

According to some specific embodiments of the present invention, the final screening of the covariates comprises:

establishing a full model by using a forward method on the basis of the final foundation model, and then establishing the population pharmacokinetic model by using a backward method on the basis of the full model, wherein

the forward method comprises: sequentially adding each covariate to a preliminary structural model of the final foundation model in step (5), wherein with a log likelihood ratio test, if an objective function value is decreased by more than 6.63 after adding 1 covariate, the newly added covariate is considered significant, with $p < 0.01$; and firstly adding the covariate having the most significant influence (the more the objective function value decreases, the more significant the influence is, and the most significant influence means that the covariate has the largest decrease in the objective function value) on the basis of the preliminary structural model to form an improved model, then testing the statistically significant covariate screened in the previous step with the improved model, and repeating the process until no significant covariates can be found; and

the backward method comprises: a process of deleting the covariates one by one on the basis of the full model, wherein if an objective function value is increased by more than 10.83 after deleting 1 covariate, the deleted covariate is considered significant, with $p < 0.001$.

The forward method: In order to establish a full model, covariates are sequentially added to the foundation model by using a stepwise forward addition method. The covariate having the most significant influence is firstly added on the basis of the structural model to form an improved model, then the statistically significant covariate screened in the previous step is tested in the improved model, and the process is repeated until no significant covariates can be found. In the forward method, if there are highly correlated covariates, covariates of clinical significance are investigated firstly according to the actual situation, and the covariates highly correlated therewith are investigated in the last step.

The backward method: This method is a process of deleting the covariates one by one on the basis of the full model.

According to some specific embodiments of the present invention, step (7) comprises evaluating the population pharmacokinetic model by using one or a combination of two or more of the following methods: a model goodness-of-fit **(GOF)** diagnostic plot, a visual predictive check (pcVPC), bootstrap and shrinkage.

The bootstrap is a resampling technique, i.e., nonparametric bootstrap, to evaluate the stability of the model.

According to some specific embodiments of the present invention,

the model goodness-of-fit diagnostic plot comprises one or a combination of two or more of the following figures: a relation diagram of a population predicted concentration (PRED) and an observed concentration (DV), a relation diagram of an individual predicted concentration (IPRED) and an observed concentration, a relation diagram of a conditional weighted residual (CWRES) and a population predicted concentration, and a relation diagram of a conditional weighted residual and time after first administration, wherein

the visual predictive check (prediction-corrected visual predictive check) comprises simulating 1000 trials according to final model parameters, covariates and actual dosages of administration, and comparative mapping of a predicted result and a measured value so as to evaluate whether the population pharmacokinetic model can well describe a plasma drug concentration-time curve of a propofol derivative;

the bootstrap comprises repeatedly fitting with population pharmacokinetic model until 1000 data sets for bootstrap replication, and randomly selecting subject data and covariates (including concentrations, time points, administration history and all covariates) for replacement to achieve the replication (the number of subjects in each data set is the same as that in the original data set); and

the shrinkage comprises estimating individual parameter values of a subject by using a Bayesian estimation method with the population pharmacokinetic model, and calculating interindividual variations and individual residuals from model predictions and observations.

The visual predictive check can be used to compare the consistency of the observations and the model-predicted median of the plasma drug concentration-time curve and the distribution range (2.5th to 97.5th percentile).

The shrinkage is used to evaluate the interindividual variations (S2) and residuals ($\varepsilon$) of the final model, and quantify the individual parameter values and random error estimates. If the shrinkage values of $\omega$ and $\varepsilon$ are large, such as > 30%, the Bayesian estimation method should be considered carefully.

According to some specific embodiments of the present invention, the shrinkage comprises evaluating the interindividual variations and residuals of the pharmacokinetic parameters by using the following equation, and quantifying the individual parameter values and random error estimates:

$$\eta_{shrinkage} = 1 - \frac{SD\left(\hat{\eta}_{ph}\right)}{\omega}$$

$$\varepsilon_{shrinkage} = 1 - SD(IWRES)$$

wherein $\eta_{shrinkage}$ is interindividual variation, $\varepsilon_{shrinkage}$ is an individual residual, $\omega$ is interindividual variation degree of individual parameter values estimated by the population pharmacokinetic model, $\eta_{ph}$ is $\eta$ value of the parameter for all individuals, IWRES is an individual weighted residual; and SD represents standard deviation.

According to some specific embodiments of the present invention, step (7) further comprises estimating individual PK parameters of the subject by using a Bayesian post-hoc method, simulating a plasma drug concentration-time curve of intravenous infusion according to actual dosages of administration, and calculating the area under the plasma drug concentration-time curve from 0-1 min (AUC 0-1 min), the area under the plasma drug concentration-time curve from 0-2 min (AUC 0-2 min), the area under the plasma drug concentration-time curve from 0-4 min (AUC 0-4 min), the area under the plasma drug concentration-time curve from 0-10 min (AUC 0-10 min), the area under the plasma drug concentration-time curve from 0-24 h (AUC 0-24 h) and the peak concentration (Cmax).

According to some specific embodiments of the present invention, clinical trials included in the analysis of the present invention comprise:

propofol derivative SAD: a phase I ramp-up trial of single intravenous injection of placebo and positive drug control in Australian healthy subjects;
propofol derivative SAD_02: a phase I ramp-up trial of single intravenous injection of positive drug control in Australian healthy subjects;
propofol derivative SAD_03: a safety and tolerance trial of single intravenous injection plus 30 min continuous intravenous infusion in Australian subjects;
propofol derivate-101: a single-center, open-label, uncontrolled phase I ramp-up trial for evaluating the single intravenous injection of propofol derivative injectable emulsion in Chinese healthy subjects;
propofol derivate-103: a single-center, open-label, randomized, two-phase, crossover study for evaluating the interaction of propofol derivative injectable emulsion and rifampin capsules (DDI) in Chinese healthy subjects;
propofol derivate-202: a multi-center, open-label, non-randomized, positive-control, dose-ramping phase IIa clinical study for evaluating the tolerance, efficacy and safety of propofol derivative injectable emulsion for induction of general anesthesia in patients undergoing elective surgery; and
propofol derivate-302: a multi-center, randomized, double-blind, propofol-controlled, parallel-group phase III study for evaluating the efficacy and safety of the propofol derivative in induction of general anesthesia in Chinese subjects undergoing elective surgery as compared with propofol.

According to some specific embodiments of the present invention, when at least one well-recorded administration time and a corresponding plasma concentration are collected from a subject after administration, the subject can be included in the population pharmacokinetic analysis.

According to some specific embodiments of the present invention, the method of the present invention is a method for determining a population pharmacokinetic model of propofol derivative injectable emulsion.

According to some specific embodiments of the present invention, the components of the propofol derivative injectable emulsion comprise: soybean oil, glycerol, triglyceride, egg yolk lecithin, sodium oleate and sodium hydroxide (see WO/2016/034079).

According to some specific embodiments of the present invention, the PopPK analysis method is based on the Guidance for Industry: Population Pharmacokinetics issued by the U.S. Food and Drug Administration (FDA) and the Guideline on Reporting the Results of Population Pharmacokinetic Analysis issued by the Committee for Medicinal Products for Human Use (CHMP).

According to some specific embodiments of the present invention, the PopPK analysis method uses a nonlinear mixed effect model (NONMEM) method to estimate typical values of the parameters and variations thereof.

According to some specific embodiments of the present invention, the PopPK analysis software used in the present invention is NONMEM 7, version 7.4.0 (ICON Development Solutions. Ellicott City, Maryland, USA); and Perl Speaks NONMEM (PsN), version 3.2.12 (Uppsala University, Sweden).

The structural models of the present invention are ultimately selected from a three-compartment model with zero-order absorption and first-order linear elimination from the central compartment, as shown in Figure 1. The PopPK model is composed of the following parameters: central compartment clearance (CL), volume of distribution in the central compartment (V1), volumes of distribution in the peripheral compartments (V2, V3), and intercompartmental clearances (Q2, Q3).

$$\frac{dX_1}{dt} = R_0 - (k + k_{12} + k_{13}) \cdot X_1 + k_{21} \cdot X_2 + k_{31} \cdot X_3$$

$$\frac{dX_2}{dt} = k_{12} \cdot X_1 - k_{21} \cdot X_2$$

$$\frac{dX_3}{dt} = k_{13} \cdot X_1 - k_{31} \cdot X_3$$

| | | | |
|---|---|---|---|
| | | $X_1$ | = Drug amount in the central compartment |
| $R_0$ | = Infusion rate | $X_2$ | = Drug amount in the peripheral compartment 1 |
| k | = Emination rate constant k =$CL/V_1$ | $X_3$ | = Drug amount in the peripheral compartment 2 |
| $k_{12}$ | = Intercompartmental rate constant between the central compartment and the peripheral compartment 1 $k_{12}$ =$Q_2/V_1$ | CL | = Central compartment clearan |
| $k_{21}$ | = Intercompartmental rate constant between the peripheral compartment 1 and the central compartment $k_{21}$ =$Q_2/V_2$ | $V_1$ | = Volume of distribution in the central compartment |
| $k_{13}$ | = Intercompartmental rate constant between the central compartment and the peripheral compartment 1 $k_{13}$ =$Q_3/V_1$ | $Q_2$, $Q_3$ | = Intercompartmental clearance |
| $k_{31}$ | = Intercompartmental rate constant between the peripheral compartment 1 and the central compartment $k_{31}$ =$Q_3/V_3$ | $V_2$, $V_3$ | = Volume of distribution in the peripheral compartment |

The parameters are estimated by using the FOCEI method of NONMEM. The correlation between random effects in the PK base model is estimated by using a diagonal S2 matrix. On the basis of the model diagnostics, a proportional model is selected as a residual model.

According to some specific embodiments of the present invention, the pre-screening results of step (5) show that: the following covariates all have a significant influence ($p < 0.01$) on the PK parameters, are included in a covariate model screening process, and investigated by using a forward method and a backward method:

(1) central compartment clearance CL: creatinine clearance (CLCR), total protein (TP), total bilirubin (TBEL), weight (WT), age (AGE), race (RACE) and blood collection site (SITE);
(2) volume of distribution in the central compartment V1: age (AGE), race (RACE) and blood collection site (SITE).

According to some specific embodiments of the present invention, a stepwise forward addition method (the forward method) is used for the PopPK model in NONMEM. The results show that WT, TP and SITE have a significant influence ($p < 0.01$) on CL, AGE has a significant influence ($p < 0.01$) on V1, and it is not found that RACE has a significant influence on CL or V1 by investigating RACE on this basis. By using a stepwise backward elimination method (the backward method), no influence factors are eliminated ($\Delta$-2LL > 10.83).

In another aspect, the present invention further provides a system for determining individual administration parameters of a compound of formula (I) or propofol.

The administration parameters can comprise individual administration data, specifically, such as dosage of administration. More specifically, the dosage of administration can comprise single dosage of administration, daily dosage of administration, administration time, administration frequency, etc. of an individual.

According to some specific embodiments of the present invention, the system for determining the individual administration parameters of the compound of formula (I) comprises a data acquisition device, a data processing device and a result output device,

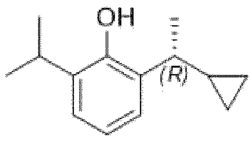
formula (I)

wherein the data comprise baseline demographic characteristic data, blood biochemical index data and blood collection site information data; with the data processing device, individual administration parameter results of the compound of formula (I) are obtained by using the following equation:

$$CL_i = \exp(4.20+0.349 \cdot \log(WT / 63.9) - 0.749 \cdot \log(TP / 72.4) + 0.238 \cdot SITE + \eta_{CL,i})$$

;

wherein CLi represents central compartment clearance of the ith subject; when collecting a sample from venous blood, SITE = 0, and when collecting a sample from artery blood, SITE = 1; WT represents weight; TP represents total protein; $\eta_{CL,i}$ is interindividual variation of the CL of the ith subject; and η follows a normal distribution with mean 0 and variance ω2, wherein the ω2 is a diagonal element of a variance-covariance matrix S2 of the interindividual variation.

According to some specific embodiments of the present invention, the result output device is used to output individual administration parameter results.

According to some specific embodiments of the present invention, with the data processing device, individual administration parameter results of the compound of formula (I) are obtained by using the following equation:

$$CL_i = \exp(4.20+0.349 \cdot \log(WT / 63.9) - 0.749 \cdot \log(TP / 72.4) + 0.238 \cdot SITE + \eta_{CL,i})$$

$$V_{1i} = \exp(0.908 + 0.426 \cdot \log(AGE / 27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

;

wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and η represents interindividual variation of a corresponding parameter.

According to some specific embodiments of the present invention, the system for determining the individual administration parameters of propofol comprises a data acquisition device, a data processing device and a result output device, wherein the data comprise baseline demographic characteristic data, blood biochemical index data and blood collection site information data; with the data processing device, individual administration parameter results of propofol are obtained by using the following equation:

$$CL_i = \exp(4.56 + \eta_{CL,i})$$

wherein CLi represents central compartment clearance of the ith subject; $\eta_{CL,i}$ is interindividual variation of the CL of the ith subject; and η follows a normal distribution with mean 0 and variance ω2, wherein the ω2 is a diagonal element of a variance-covariance matrix S2 of the interindividual variation.

According to some specific embodiments of the present invention, with the data processing device, individual administration parameter results of propofol are obtained by using the following equation:

$$CL_i = \exp(4.56 + \eta_{CL,i})$$

$$V_{1i} = \exp(2.25 + \eta_{V_1,i})$$

$$Q_{2i} = \exp(4.96)$$

$$V_{2i} = \exp(3.63)$$

$$Q_{3i} = \exp(3.88)$$

$$V_{3i} = \exp(5.57)$$

wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and η represents interindividual variation of a corresponding parameter.

According to some specific embodiments of the present invention, the result output device is used to output individual administration parameter results.

The methods not specified in the present application can be implemented with reference to conventional methods in the field.

In conclusion, the present invention provides a method and system for determining a population pharmacokinetic model of a propofol derivative and propofol. The method of the present invention has the following advantages:
The three-compartment linear pharmacokinetic model can well describe the pharmacokinetic characteristics in the dosage range in this study. After model evaluation, it shows that the final model has relatively good stability, accuracy and good prediction performance, no obvious deviation is observed in the diagnostic plot, and the pcVPC result shows that the model can fully reproduce the central tendency and variability of the original data. The population pharmacokinetic model of the propofol derivative and propofol established by the method of the present invention can be used for guiding the dosage regimen in the following clinical trials (phase I, phase II or phase III).

The weight and the total protein show a significant influence on the CL of the drug. The weight in the range of 45 kg and 90 kg shows a relatively small influence (the relative median changes are -11.6% and 12.7%, respectively). The influence of the total protein (the relative median changes are 6.8% and -6.3%, respectively) is expected to be clinically insignificant. CL estimated by the model is also significantly influenced by different blood collection sites. The age has a significant influence on the V1 of the drug, and the results in the trials show that the age in the range of 19.8 to 53 years old has no influence on AUC0-24 and has an influence on Cmax, but is expected to be clinically insignificant.

## Brief Description of the Drawings

Figure 1 shows a structural diagram of a three-compartment model of the present invention;
Figure 2 shows diagnostic plots of a final drug model in example 1 of the present invention;
Figure 3 shows distribution diagrams of interindividual variations (ETA) of the final model of the drug in example 1 of the present invention;
Figure 4 shows prediction-corrected visual predictive check (pcVPC) plots of the drug in example 1 of the present invention;
Figure 5 shows diagnostic plots of a final drug model in example 2 of the present invention;
Figure 6 shows prediction-corrected visual predictive check (pcVPC) plots of the drug in example 2 of the present invention;
Figure 7 shows a plasma drug concentration-time relational plot of the drug in example 1 of the present invention;
Figure 8 shows a plasma drug concentration-time relational plot of the drug in example 2 of the present invention.

Detailed Description of Embodiments

The technical solutions of the present invention will be described in detail below in conjunction with the drawings and examples, but the protection scope of the present invention includes but is not limited thereto.

**Example 1**

This example provides a method for determining a population pharmacokinetic model of a propofol derivative. The method comprises:
1. Acquisition of data: Clinical trial

propofol derivative SAD: a phase I ramp-up trial of single intravenous injection of placebo and positive drug control in Australian healthy subjects;
propofol derivative SAD_02: a phase I ramp-up trial of single intravenous injection of positive drug control in Australian healthy subjects;
propofol derivative SAD_03: a safety and tolerance trial of single intravenous injection plus 30 min continuous intravenous infusion in Australian subjects;
propofol derivate-101: a single-center, open-label, uncontrolled phase I ramp-up trial for evaluating the single intravenous injection of propofol derivative injectable emulsion in Chinese healthy subjects;
propofol derivate-103: a single-center, open-label, randomized, two-phase, crossover study for evaluating the interaction of propofol derivative injectable emulsion and rifampin capsules (DDI) in Chinese healthy subjects;
propofol derivate-202: a multi-center, open-label, non-randomized, positive-control, dose-ramping phase IIa clinical study for evaluating the tolerance, efficacy and safety of propofol derivative injectable emulsion for induction of general anesthesia in patients undergoing elective surgery; and
propofol derivate-302: a multi-center, randomized, double-blind, propofol-controlled, parallel-group phase III study for evaluating the efficacy and safety of the propofol derivative in induction of general anesthesia in Chinese subjects undergoing elective surgery as compared with propofol.

2. Determination of data included in an analysis:
The sampling scheme is as shown in Table 1 below:

Table 1. Sampling scheme

| Scheme no. | Dosage | Planned sampling design |
|---|---|---|
| Propofol derivative SAD | 0.128 mg/kg<br>0.192 mg/kg<br>0.288 mg/kg<br>0.432 mg/kg<br>0.54 mg/kg<br>0.648 mg/kg<br>0.81 mg/kg | Arterial blood samples were collected within 30 min (0 min) before injection and at 0.5 min, 1 min, 1.5 min, 2 min, 2.5 min, 3 min, 3.5 min, 4 min, 5 min, 6 min, 8 min, 12 min, 15 min, 30 min, 60 min, 90 min, 2 hr, 3 hr and 4 hr after the start of injection; and venous blood samples were collected at 6 hr, 8 hr and 24 hr after the start of injection. |

(continued)

| Scheme no. | Dosage | Planned sampling design |
|---|---|---|
| Propofol derivative SAD_02 | 0.288 mg/kg<br>0.432 mg/kg<br>0.54 mg/kg<br>0.648 mg/kg<br>0.81 mg/kg | Arterial blood samples were collected within 30 min (0 min) before injection and at 0.5 min, 1 min, 1.5 min, 2 min, 2.5 min, 3 min, 3.5 min, 4 min, 5 min, 6 min, 8 min, 12 min, 15 min, 30 min, 60 min, 90 min, 2 hr, 3 hr and 4 hr after the start of injection; and venous blood samples were collected at 6 hr, 8 hr and 24 hr after the start of injection. |
| Propofol derivative SAD_03 | 0.288 mg/kg + 1 mg/kg/h<br>0.540 mg/kg + 2 mg/kg/h | Arterial blood samples were collected within 30 min (0 min) before injection and at 0.5 min, 1 min, 1.5 min, 2 min, 2.5 min, 3 min, 3.5 min, 4 min, 5 min, 8 min, 11 min, 21 min, 25 min, 31 min, 32 min, 34 min, 36 min, 40 min, 50 min, 60 min, 90 min, 2 hr, 3 hr and 4 hr after the start of first injection; and venous blood samples were collected at 6 hr, 8 hr and 24 hr after the start of first injection. |
| Propofol derivate-101 | 0.15 mg/kg<br>0.4 mg/kg<br>0.6 mg/kg<br>0.9 mg/kg | Venous blood samples were collected within 30 min (0 min) before injection, at 0.5 min, 1 min, 2 min, 3 min, 5 min, 8 min, 15 min, 30 min, 60 min, 90 min, 2 hr, 3 hr, 4 hr, 6 hr, 8 hr and 24 hr after the start of injection, and at time points when the verbal response disappeared and recovered. |
| Propofol derivate-103 | 0.4 mg/kg<br>0.4 mg/kg + rifampin | Arterial blood samples were collected within 30 min (0 min) before injection and at 1 min, 2 min, 4 min, 8 min, 15 min, 30 min and 60 min after the start of injection; and venous blood samples were collected at 2 hr, 3 hr, 4 hr, 6 hr, 8 hr, 12 hr and 24 hr after the start of injection. |
| Propofol | 0.3 mg/kg | Only venous blood samples were collected within 30 min (0 |
| derivate-202 | 0.4 mg/kg<br>0.5 mg/kg | min) before injection and at 0.5 min, 1 min, 2 min, and 3 min after the start of injection, except for 4 cases in which both arterial and venous blood samples were collected. |
| Propofol derivate-302 | Propofol derivatives:<br>0.4 mg/kg (first time)<br>0.2 mg/kg (supplementing if necessary) | Venous blood samples were collected within 30 min (0 min) before injection and at 5 min, 15 min and 60 min after the start of injection. |

The original data set comprises 2609 measurable plasma drug concentration data from 219 subjects (wherein 81 people receive both arterial and venous blood collection, and subjects receiving another blood collecting method are regarded as new individuals, that is, 300 individuals are included in the population analysis).

A pharmacokinetic data set included in the analysis are determined by evaluating the clinical trial data included:

plasma drug concentration (see Figure 7);
baseline demographic characteristic data: race, age, height, weight and gender;
blood biochemical index data: blood total protein content, creatinine clearance, glutamic-oxalacetic transaminase and glutamic-pyruvic transaminase; and
blood collection sites.

3. Processing of data:

Processing the data in step (2) comprising:

determining and processing the observations below the lower limit of detection comprising: determining the lower limit of detection by a detecting instrument, wherein the observations below the lower limit of detection are not used for population pharmacokinetic analysis, and if the proportion of the observations below the lower limit of detection is greater than 15%, investigating the influence of the observations below the lower limit of detection on model goodness-of-fit and modeling parameters by using a likelihood function method;
determining and processing the anomalous value data comprising: checking whether there are anomalous values in a sample according to a plasma concentration - time curve in subjects receiving drug administration, and eliminating

the anomalous values;
determining and processing the outliers comprising: determining outliers according to residual analysis of preliminary modeling results, and eliminating the outliers; and
processing the missing covariates comprising: if the missing covariate for the subject is less than 15%, applying imputation with the median of the data set for continuous covariates, and applying imputation with a value of the most common category for categorical covariates; and if the missing covariate for the subject is greater than 15%, applying no imputation, and performing exploratory analysis on the PK parameters of the subject with full covariate information by using a Bayesian estimation method.

4. Establishment of a preliminary population pharmacokinetic foundation model:

Establishing the preliminary foundation model on the basis of the data processed in step 3 comprising:

(1) describing interindividual variation of the PK parameters by using the following equation:

$$\theta_i = \exp(\theta_T + \eta_i);$$

(2) describing variability of a residual by using the following equation:

$$log(y_{ij}) = log(\hat{y}_{ij}) + \varepsilon_{ij};$$

(3) comparing a one-compartment model, a two-compartment model and a three-compartment model by comparing whether the decrease of the objective function values of the two nested models is significant on the basis of the plasma drug concentration-time curve, and selecting the three-compartment model as shown in Figure 1 as the preliminary foundation model, wherein CL represents central compartment clearance; V1 represents volume of distribution in the central compartment; V2 represents volume of distribution in the peripheral compartment 1; V3 represents volume of distribution in the peripheral compartment 2; Q2 represents intercompartmental clearance between the peripheral compartment 1 and the central compartment; Q3 represents intercompartmental clearance between the peripheral compartment 2 and the central compartment; R0 represents infusion rate; and K represents elimination rate constant.

5. Establishment of a final population pharmacokinetic foundation model:

Establishing the final foundation model on the basis of the preliminary foundation model in step 4 comprising:

(1) determining covariates included in the evaluation on the basis of clinical knowledge and a drug action mechanism:

baseline demographic characteristic covariates: baseline values for age, gender, weight and race;
blood biochemical index covariates: creatinine clearance, total protein, glutamic-oxalacetic transaminase, glutamic-pyruvic transaminase, alkaline phosphatase and total bilirubin; and
blood collection sites.

(2) establishing a final population pharmacokinetic foundation model on the basis of the covariates included in the evaluation.

6. Establishment of the population pharmacokinetic model:

Establishing the population pharmacokinetic model by adding the screened covariates on the basis of the final foundation model in step 5 comprising:

(1) Pre-screening of covariates:

analyzing the correlation between the continuous covariates and the PK parameters by using the following equation:

$$\theta_i = \theta_{pop} \cdot \left( \frac{Cov_i}{Cov_{pop}} \right)^{k_{cov}};$$

and analyzing the correlation between the categorical covariates and the PK parameters by using the following equation:

$$\theta_i = \theta_{pop} \times \exp(k_{cov} \cdot X_i)$$

(2) Final screening of the covariates by using a forward method and a backward method, and establishing the population pharmacokinetic model:

The forward method: sequentially adding each covariate to a preliminary structural model of the final foundation model in step (5), wherein with a log likelihood ratio test, if an objective function value is decreased by more than 6.63 after adding 1 covariate, the newly added covariate is considered significant, with $p < 0.01$; and firstly adding the covariate having the most significant influence on the basis of the preliminary structural model to form an improved model, then testing the statistically significant covariate screened in the previous step with the improved model, and repeating the process until no significant covariates can be found to obtain a full model;

The backward method: a process of deleting the covariates one by one on the basis of the full model, wherein if an objective function value is increased by more than 10.83 after deleting 1 covariate, the deleted covariate is considered significant, with $p < 0.001$.

A stepwise forward addition method is used for the propofol derivative PopPK model in NONMEM. The results show that WT, TP and SITE have a significant influence ($p < 0.01$) on CL, AGE has a significant influence ($p < 0.01$) on $V_1$, and it is not found that RACE has a significant influence on CL or $V_1$ by investigating RACE on this basis. By using a stepwise backward elimination method, no influence factors are eliminated ($\Delta_{-2LL} > 10.83$). A stepwise forward addition method is used for the propofol PopPK model in NONMEM. The results show that WT has a significant influence ($p < 0.01$) on CL. The influence of WT on the CL of the propofol derivative is eliminated by using a stepwise backward elimination method ($\Delta$-2LL = 8.691).

(3) Establishing the model

$$CL_i = \exp(4.20 + 0.349 \cdot \log(WT / 63.9) - 0.749 \cdot \log(TP / 72.4) + 0.238 \cdot SITE + \eta_{CL,i})$$

$$V_{1i} = \exp(0.908 + 0.426 \cdot \log(AGE / 27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

In the final PopPK model of the propofol derivative, the weight and the total protein have a significant influence on the CL, and CL estimated by the model is also significantly influenced by different blood collection sites; and the age has an influence on $V_1$.

7. Evaluation of the population pharmacokinetic model:

Evaluating the pharmacokinetic model established in step 6 comprising:

(1) Model goodness-of-fit **(GOF)** diagnostic plot

The diagnostic plots of the final PopPK model for the drug are as shown in Figure 2. The results show that there is good consistency between an observed concentration value and a predicted concentration, and no significant deviations are observed in the plots of conditional weighted residuals versus time and versus a predicted concentration. In Figure 2, the upper left panel is a diagnostic plot of observations versus individual predictions in the final model of the drug, the upper right panel is a diagnostic plot of observations versus population predictions in the final model of the drug, the lower left panel is a diagnostic plot of conditional weight residuals (CWRES) versus time, and the lower right panel is a diagnostic plot of CWRES versus population predictions, wherein solid lines in the middle are standard lines of 0, and dotted lines are auxiliary lines of |CWRES| = 5.

(2) Distribution of interindividual random effects (ETA) of the PopPK final model of the drug is as shown in Figure 3. The results show that the ETA is generally distributed symmetrically around 0. In Figure 3, the vertical coordinate is frequency, etaCL is interindividual variation of CL, etaV1 is interindividual variation of V1, etaQ2 is interindividual variation of Q2, etaQ3 is interindividual variation of Q3, etaV3 is interindividual variation of V3, and bold lines in the middle are standard lines of 0.

(3) Prediction-corrected visual predictive check **(pcVPC)**

pcVPC is used to evaluate the ability of the model to reproduce the data distribution. 1000 trials are simulated and reproduced according to the observed covariate information of each subject, the estimated values of the final population pharmacokinetic model parameters, the random effects and the residual errors. The pcVPC of the plasma drug concentration-time curve is as shown in Figure 4. Figure 4 shows the observed concentrations (dots) of all subjects, the model-predicted 95% confidence (middle shaded area) interval for the median and the model-predicted 95% confidence intervals (upper and lower shaded areas) for the 2.5th and 97.5th percentiles. The results show that the final population pharmacokinetic model can fully predict the central tendency and variability of drug concentrations of all subjects in the clinical study. In Figure 4, the circles are observations, the middle solid lines are medians of the observations, the upper and lower dashed lines correspond to the 97.5th and 2.5th percentiles respectively, the middle shaded area is the model-predicted 95% confidence interval for the median, the upper and lower shaded areas are 95% confidence intervals for the median lines and the 2.5th and 97.5th percentile lines for data of the 1000 simulations, and data lower than the lower limit of detection are not shown in this figure.

(4) Bootstrap

See Table 2 below for the comparison between the parameters estimated by the final model and the parameters estimated by Bootstrap. The median of the parameters estimated by Bootstrap is similar to the median of the parameters estimated by the final PopPK model. 95% CI of the parameters estimated by the final PopPK model is highly overlapped with 95% CI of the parameters estimated by Bootstrap (represented by the 2.5th to 97.5th percentile interval), indicating that the final model has relatively good stability and accuracy.

Table 2. Parameters estimated by final model and parameters estimated by Bootstrap

| Parameters | Parameter description | Typical values of population model parameters (95% CI) | Median of Bootstrap population model parameters ($2.5^{th}$ to $97.5^{th}$ percentiles) |
|---|---|---|---|
| $exp(\theta_1)$ | Clearance, CL (L/hr) | 66.4 (63.5 to 69.2) | 66.4 (63.7 to 69.4) |
| $exp(\theta_2)$ | Volume of distribution in the central compartment, $V_1$ (L) | 2.48 (2.25 to 2.71) | 2.48 (2.27 to 2.74) |
| $exp(\theta_3)$ | Intercompartmental clearance between the central compartment and the peripheral compartment 1, $Q_2$ (L/hr) | 58 (50.8 to 65.1) | 57.9 (51.5 to 65.6) |
| $exp(\theta_4)$ | Volume of distribution in the peripheral compartment 1, $V_2$ (L) | 5.77 (4.96 to 6.58) | 5.74 (5.07 to 6.7) |

(continued)

| Parameters | Parameter description | Typical values of population model parameters (95% CI) | Median of Bootstrap population model parameters ($2.5^{th}$ to $97.5^{th}$ percentiles) |
|---|---|---|---|
| $exp(\theta_5)$ | Clearance between the central compartment and the peripheral compartment 2, $Q_3$ (L/hr) | 59.0 (54.5 to 63.5) | 59.1 (54.6 to 63.6) |
| $exp(\theta_6)$ | Volume of distribution in the peripheral compartment 2, $V_3$ (L) | 78.3 (72.8 to 83.8) | 78.4 (73 to 83.9) |
| $\theta_7$ | Influence of weight on clearance, WT on CL | 0.349 (0.21 to 0.488) | 0.348 (0.206 to 0.489) |
| $\theta_8$ | Influence of age on volume of distribution in the central compartment, AGE on $V_1$ | 0.426 (0.194 to 0.659) | 0.441 (0.172 to 0.66) |
| $\theta_9$ | Influence of total protein on clearance, TP on CL | -0.749 (-1.21 to - 0.285) | -0.743 (-1.22 to - 0.259) |
| $exp(\theta_{10})$ | Influence of arterial blood collection on clearance, SITE on | 1.27 (1.2 to 1.34) | 1.27 (1.2 to 1.33) |
| | CL | | |
| $\omega_{CL}$ | Interindividual variation of clearance, CL% | 14.5 (12 to 16.9) | 14.3 (11.9 to 17) |
| $\omega_{V1}$ | Interindividual variation of volume of distribution in the central compartment, $V_1$% | 41.4 (28 to 54.9) | 40.4 (27.7 to 54.5) |
| $\omega_{Q2}$ | Interindividual variation of intercompartmental clearance between the central compartment and the peripheral compartment 1, $Q_2$% | 28.9 (23.1 to 34.7) | 29.0 (23.1 to 35.5) |
| $\omega_{Q3}$ | Interindividual variation of intercompartmental clearance between the central compartment and the peripheral compartment 2, $Q_3$% | 29.1 (23.1 to 35.1) | 28.8 (22.8 to 36.3) |
| $\omega_{V3}$ | Interindividual variation of volume of distribution in the peripheral compartment 2, $V_3$% | 30.7 (25.6 to 35.8) | 30.5 (25.3 to 35.7) |
| $\sigma_1$ | Residual of venous blood collection (%) | 30.6 (26.6 to 34.5) | 30.5 (26.3 to 34.6) |
| $\sigma_2$ | Residual of arterial blood collection (%) | 16.9 (15.8 to 18.1) | 16.9 (15.7 to 18.2) |

(4) **Shrinkage**

The shrinkage values of the final model parameters are as shown in Table 3.

In the final population pharmacokinetic model, the shrinkage amplitudes of $\omega_{V1}$, $\omega_{Q2}$, $\omega_{Q3}$, and $\omega_{V3}$ are slightly higher than 30%.

Table 3. Shrinkage values of final model parameters

| Parameters | Parameter description | Shrinkage values (%) |
|---|---|---|
| ωCL | Clearance, CL% | 29.2% |
| ωV1 | Volume of distribution in the central compartment, V1% | 36.5% |
| ωQ2 | Intercompartmental clearance between the central compartment and the peripheral compartment 1, Q2% | 44.6% |
| ωQ3 | Intercompartmental clearance between the | 35.5% |
| | central compartment and the peripheral compartment 2, Q3% | |
| ωV3 | Volume of distribution in the peripheral compartment 2, V3% | 32.9% |
| σ1 | Residual of venous blood collection (%) | 17.3% |

(continued)

| Parameters | Parameter description | Shrinkage values (%) |
|---|---|---|
| σ2 | Residual of arterial blood collection (%) | 13.2% |

Conclusion:

The three-compartment linear pharmacokinetic model can well describe the pharmacokinetic characteristics of the propofol derivative in the dosage range in this study. After model evaluation, it shows that the final model has relatively good stability, accuracy and good prediction performance.

The weight and the total protein show a significant influence on the CL of the propofol derivative. The weight in the range of 45 kg and 90 kg shows a relatively small influence (the relative median changes are -11.6% and 12.7%, respectively). The influence of the total protein (the relative median changes are 6.8% and -6.3%, respectively) is expected to be clinically insignificant. CL estimated by the model is also significantly influenced by different blood collection sites. The age has a significant influence on the $V_1$ of the propofol derivative, and the results in the propofol derivative-302 trial show that the age in the range of 19.8 to 53 years old has no influence on $AUC_{0-24}$ and has an influence on Cmax, but is expected to be clinically insignificant.

**Example 2**

This example provides a method for determining a population pharmacokinetic model of propofol. The method comprises:

1. Acquisition of data: Clinical trial
a multi-center, randomized, double-blind, propofol-controlled, parallel-group phase III study for evaluating the efficacy and safety of propofol in induction of general anesthesia in Chinese subjects undergoing elective surgery.
2. Determination of data included in an analysis:
The sampling scheme is as shown in Table 4 below:

Table 4. Propofol sampling scheme

| Scheme no. | Dosage | Planned sampling design |
|---|---|---|
| Propofol | Propofol: | Venous blood samples were collected within |
| | 2.0 mg/kg (first time) 1.0 mg/kg (supplementing if necessary) | 30 min (0 min) before injection and at 5 min, 15 min and 60 min after the start of injection. |

82 measurable plasma drug concentration data from 28 subjects (60 of 88 subjects treated with propofol in the trial further receive propofol treatment for other purposes after induced anesthesia, and are not included in the analysis) are included in the propofol pharmacokinetic analysis data set.

A pharmacokinetic data set included in the analysis are determined by evaluating the clinical trial data included:

plasma drug concentration data: acquired by means of implementation of the sampling scheme (see Figure 8);
baseline demographic characteristic data: race, age, height, weight and gender;
blood biochemical index data: blood total protein content, creatinine clearance, glutamic-oxalacetic transaminase and glutamic-pyruvic transaminase; and
blood collection sites.

3. Processing of data:

Processing the data in step (2) comprising:

determining and processing the observations below the lower limit of detection comprising: determining the lower limit of detection by a detecting instrument, wherein the observations below the lower limit of detection are not used for population pharmacokinetic analysis, and if the proportion of the observations below the lower limit of detection is greater than 15%, investigating the influence of the observations below the lower limit of detection on model goodness-of-fit and modeling parameters by using a likelihood function method;
determining and processing the anomalous value data comprising: checking whether there are anomalous values

in a sample according to a plasma concentration - time curve in subjects receiving drug administration, and eliminating the anomalous values;
determining and processing the outliers comprising: determining outliers according to residual analysis of preliminary modeling results, and eliminating the outliers; and
processing the missing covariates comprising: if the missing covariate for the subject is less than 15%, applying imputation with the median of the data set for continuous covariates, and applying imputation with a value of the most common category for categorical covariates; and if the missing covariate for the subject is greater than 15%, applying no imputation, and performing exploratory analysis on the PK parameters of the subject with full covariate information by using a Bayesian estimation method.

4. Establishment of a preliminary population pharmacokinetic foundation model:
Establishing the preliminary foundation model on the basis of the data processed in step 3 comprising:

(1) describing interindividual variation of the PK parameters by using the following equation:

$$\theta_i = \exp(\theta_T + \eta_i),$$

(2) describing variability of a residual by using the following equation:

$$log\left(y_{ij}\right) = log\left(\hat{y}_{ij}\right) + \varepsilon_{ij},$$

(3) comparing a one-compartment model, a two-compartment model and a three-compartment model by comparing whether the decrease of the objective function values of the two nested models is significant on the basis of the plasma drug concentration-time curve, and selecting the three-compartment model as shown in Figure 1 as the preliminary foundation model, wherein CL represents central compartment clearance; V1 represents volume of distribution in the central compartment; V2 represents volume of distribution in the peripheral compartment 1; V3 represents volume of distribution in the peripheral compartment 2; Q2 represents intercompartmental clearance between the peripheral compartment 1 and the central compartment; Q3 represents intercompartmental clearance between the peripheral compartment 2 and the central compartment; R0 represents infusion rate; and K represents elimination rate constant.

5. Establishment of a final population pharmacokinetic foundation model:
Establishing the final foundation model on the basis of the preliminary foundation model in step 4 comprising:

(1) determining covariates included in the evaluation on the basis of clinical knowledge and a drug action mechanism:

baseline demographic characteristic covariates: baseline values for age, gender, weight and race;
blood biochemical index covariates: creatinine clearance, total protein, glutamic-oxalacetic transaminase, glutamic-pyruvic transaminase, alkaline phosphatase and total bilirubin; and
blood collection sites.

(2) establishing a final population pharmacokinetic foundation model on the basis of the covariates included in the evaluation.

6. Establishment of the population pharmacokinetic model:
Establishing the population pharmacokinetic model by adding the screened covariates on the basis of the final foundation model in step 5 comprising:

(1) Pre-screening of covariates:

analyzing the correlation between the continuous covariates and the PK parameters by using the following equation:

$$\theta_i = \theta_{pop} \cdot \left( \frac{Cov_i}{Cov_{pop}} \right)^{k_{cov}}$$;

and analyzing the correlation between the categorical covariates and the PK parameters by using the following equation:

$$\theta_i = \theta_{pop} \times \exp(k_{cov} \cdot X_i)$$

(2) Final screening of the covariates by using a forward method and a backward method, and establishing the population pharmacokinetic model:

The forward method: sequentially adding each covariate to a preliminary structural model of the final foundation model in step (5), wherein with a log likelihood ratio test, if an objective function value is decreased by more than 6.63 after adding 1 covariate, the newly added covariate is considered significant, with $p < 0.01$; and firstly adding the covariate having the most significant influence on the basis of the preliminary structural model to form an improved model, then testing the statistically significant covariate screened in the previous step with the improved model, and repeating the process until no significant covariates can be found to obtain a full model;
The backward method: a process of deleting the covariates one by one on the basis of the full model, wherein if an objective function value is increased by more than 10.83 after deleting 1 covariate, the deleted covariate is considered significant, with $p < 0.001$.

A stepwise forward addition method is used for the propofol PopPK model in NONMEM. The results show that WT has a significant influence ($p < 0.01$) on CL. The influence of WT on the CL of the propofol is eliminated by using a stepwise backward elimination method ($\Delta_{-2LL}$ = 8.691).
In covariate screening, it is not found that covariates have a significant influence on the pharmacokinetic PK parameters of propofol.
7. Evaluation of the population pharmacokinetic model:
Evaluating the pharmacokinetic model established in step 6 comprising:

(1) Model goodness-of-fit **(GOF)** diagnostic plot
The diagnostic plots of the final PopPK model for the drug are as shown in Figure 5. The results show that there is good consistency between an observed concentration value and a predicted concentration, and no significant deviations are observed in the plots of conditional weighted residuals versus time and versus a predicted concentration. In Figure 5, the upper left panel is a diagnostic plot of observations versus individual predictions in the final model of the drug, the upper right panel is a diagnostic plot of observations versus population predictions in the final model of the drug, the lower left panel is a diagnostic plot of conditional weight residuals (CWRES) versus time, and the lower right panel is a diagnostic plot of CWRES versus population predictions, wherein solid lines in the middle are standard lines of 0, and dotted lines are auxiliary lines of |CWRES| = 5.
(2) Prediction-corrected visual predictive check **(pcVPC)**
pcVPC is used to evaluate the ability of the model to reproduce the data distribution. 1000 trials are simulated and reproduced according to the observed covariate information of each subject, the estimated values of the final population pharmacokinetic model parameters, the random effects and the residual errors. The pcVPC of the plasma drug concentration-time curve is as shown in Figure 6. Figure 6 shows the observed concentrations (dots) of all subjects, the model-predicted 95% confidence (middle shaded area) interval for the median and the model-predicted 95% confidence intervals (upper and lower shaded areas) for the 2.5th and 97.5th percentiles. The results show that the final population pharmacokinetic model can fully predict the central tendency and variability of drug concentrations of all subjects in the clinical study. In Figure 6, the circles are observations, the middle solid lines are medians of the observations, the upper and lower dashed lines correspond to the 97.5th and 2.5th percentiles respectively, the middle shaded area is the model-predicted 95% confidence interval for the median, the upper and lower shaded areas are 95% confidence intervals for the median lines and the 2.5th and 97.5th percentile lines for data of the 1000 simulations, and data lower than the lower limit of detection are not shown in this figure.
(3) Bootstrap
See Table 5 below for the comparison between the parameters estimated by the final model and the parameters estimated by Bootstrap. The median of the parameters estimated by Bootstrap is similar to the median of the

parameters estimated by the final PopPK model. 95% CI of the parameters estimated by the final PopPK model is highly overlapped with 95% CI of the parameters estimated by Bootstrap (represented by the 2.5th to 97.5th percentile interval), indicating that the final model has relatively good stability and accuracy.

Table 5. Parameters estimated by final model and parameters estimated by Bootstrap

| Parameters | Parameter description | Typical values of population model parameters (95% CI) | Median of Bootstrap population model parameters (2.5th to 97.5th percentiles) |
|---|---|---|---|
| $exp(\theta_1)$ | Clearance, CL (L/hr) | 95.2 (76.6 to 114) | 91.6 (25.1 to 114) |
| $exp(\theta_2)$ | Volume of distribution in the central compartment, $V_1$ (L) | 9.46 (4.83 to 14.1) | 8.92 (1.15 to 17.2) |
| $exp(\theta_3)$ | Intercompartmental clearance between the central compartment and the peripheral compartment 1, $Q_2$ (L/hr) | 142 (88.4 to 196) | 132 (13.0 to 200) |
| $exp(\theta_4)$ | Volume of distribution in the peripheral compartment 1, $V_2$ (L) | 37.6 (25.2 to 50.0) | 35.6 (1.64 to 53.3) |
| $exp(\theta_5)$ | Clearance between the central compartment and the peripheral compartment 2, $Q_3$ (L/hr) | 48.6, FIX(-) | 48.6, FIX(-) |
| *exp(6)* | Volume of distribution in the peripheral compartment 2, $V_3$ (L) | 262, FIX(-) | 262, FIX(-) |
| $\omega_{CL}$ | Clearance, CL% | 23.0 (14.8 to 31.1) | 22.5 (13.6 to 30.6) |
| $\omega_{V1}$ | Volume of distribution in the central compartment, $V_1$% | 30.5 (8.11 to 52.9) | 32.4 (6.02 to 49.5) |
| $\sigma_1$ | Residual (%) | 19.2 (12.7 to 25.7) | 17.1 (11 to 23.1) |

(4) **Shrinkage**

The shrinkage values of the final model parameters of propofol are as shown in Table 6. The circles are observations, the solid lines are medians of the observations, the upper and lower dashed lines correspond to the 97.5th and 2.5th percentiles respectively, the shaded area marked as 1 is the model-predicted 95% confidence interval for the median, the shaded areas marked as 2 are 95% confidence intervals for the median lines and the 2.5th and 97.5th percentile lines for data of the 1000 simulations, and data lower than the lower limit of detection are not shown in this figure. In the final population pharmacokinetic model, in the final population pharmacokinetic model of propofol, the shrinkage amplitude of $\omega_{V1}$ is slightly higher than 30%.

Table 6. Shrinkage values of final model parameters of propofol

| Parameters | Parameter description | Shrinkage values (%) |
|---|---|---|
| ωCL | Clearance, CL% | 13.2% |
| ωV1 | Volume of distribution in the central compartment, V1% | 31.3% |
| σ1 | Residual (%) | 22.8% |

Conclusion:

The three-compartment linear pharmacokinetic model can well describe the pharmacokinetic characteristics of propofol in the dosage range in this study. After model evaluation, it shows that the final model has relatively good stability, accuracy and good prediction performance.

It is not found that covariates have a significant influence on the pharmacokinetic parameters of propofol.

**Example 3**

This example provides a system for determining clinical individual administration parameters of a compound of formula (I).

The system comprises a data acquisition device, a data processing device and a result output device.

The data comprise baseline demographic characteristic data, blood biochemical index data and blood collection sites. With the data processing device, individual administration parameter (dosage) results of the compound of formula (I) are obtained by using the following equation:

$$CL_i = \exp(4.20 + 0.349 \cdot \log(WT/63.9) - 0.749 \cdot \log(TP/72.4) + 0.238 \cdot SITE + \eta_{CL,i})$$

;

$$V_{1i} = \exp(0.908 + 0.426 \cdot \log(AGE/27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

wherein CLi represents central compartment clearance of the ith subject; when collecting a sample from venous blood, SITE = 0, and when collecting a sample from artery blood, SITE = 1; WT represents weight; TP represents total protein; $\eta_{CL,i}$ is interindividual variation of the CL of the ith subject; and η follows a normal distribution with mean 0 and variance ω2, wherein the ω2 is a diagonal element of a variance-covariance matrix S2 of the interindividual variation;

wherein $V_{1i}$ represents volume of distribution in the central compartment of the ith subject;

$V_{2i}$ represents volume of distribution in the peripheral compartment 1 of the ith subject;

$V_{3i}$ represents volume of distribution in the peripheral compartment 2 of the ith subject;

$Q_{2i}$ represents intercompartmental clearance between the peripheral compartment 1 and the central compartment of the ith subject;

$Q_{3i}$ represents intercompartmental clearance between the peripheral compartment 2 and the central compartment of the ith subject;

AGE represents age; and η represents interindividual variation of a corresponding parameter.

**Example 4**

This example provides a system of individual administration parameters of propofol. The system comprises a data acquisition device, a data processing device and a result output device. With the data processing device, the individual administration parameter (dosage) results of propofol are obtained by using the following equation:

$$CL_i = \exp(4.56 + \eta_{CL,i})$$

$$V_{1i} = \exp(2.25 + \eta_{V_1,i})$$

$$Q_{2i} = \exp(4.96)$$

$$V_{2i} = \exp(3.63)$$

$$Q_{3i} = \exp(3.88)$$

$$V_{3i} = \exp(5.57)$$

wherein CLi represents central compartment clearance of the ith subject; $\eta_{CL,i}$ is interindividual variation of the CL of the ith subject; and η follows a normal distribution with mean 0 and variance ω2, wherein the ω2 is a diagonal element of a variance-covariance matrix S2 of the interindividual variation;
wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and η represents interindividual variation of a corresponding parameter.

## Claims

1. A method for determining a population pharmacokinetic model of a compound of formula (I) or propofol, wherein the method comprises determining the population pharmacokinetic model of the compound of formula (I) or propofol:

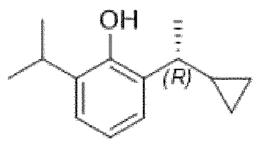 formula (I),

wherein an equation of pharmacokinetic parameters in the population pharmacokinetic model of the compound of formula (1) comprises:

$$CL_i = \exp(4.20+0.349 \cdot \log(WT / 63.9) - 0.749 \cdot \log(TP / 72.4) + 0.238 \cdot SITE + \eta_{CL,i})$$

;

an equation of pharmacokinetic parameters in the population pharmacokinetic model of propofol comprises:

$$CL_i = \exp(4.56 + \eta_{CL,i})$$

wherein CLi represents central compartment clearance of the ith subject; when collecting a sample from venous blood, SITE = 0, and when collecting a sample from artery blood, SITE = 1; WT represents weight; TP represents total protein; $\eta_{CL,i}$ is interindividual variation of the CL of the ith subject; and η follows a normal distribution with mean 0 and variance ω2, wherein the ω2 is a diagonal element of a variance-covariance matrix S2 of the interindividual variation.

2. The method according to claim 1, wherein

the equation of the pharmacokinetic parameters in the population pharmacokinetic model of the compound of formula (1) further comprises:

$$V_{1i} = \exp(0.908 + 0.426 \cdot \log(AGE / 27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

the equation of the pharmacokinetic parameters in the population pharmacokinetic model of propofol further comprises:

$$V_{1i} = \exp(2.25 + \eta_{V_1,i})$$

$$Q_{2i} = \exp(4.96)$$

$$V_{2i} = \exp(3.63)$$

$$Q_{3i} = \exp(3.88)$$

$$V_{3i} = \exp(5.57)$$

wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and $\eta$ represents interindividual variation of a corresponding parameter.

**3.** The method according to claim 1, wherein the method comprises the following steps:

(1) acquisition of data;
(2) determination of data included in an analysis;
(3) processing of data;
(4) establishment of a preliminary population pharmacokinetic foundation model;
(5) establishment of a final population pharmacokinetic foundation model;
(6) establishment of the population pharmacokinetic model; and
(7) evaluation of the population pharmacokinetic model.

**4.** The method according to claim 3, wherein the data in step (1) are derived from clinical trial data.

**5.** The method according to claim 3, wherein step (2) comprises determining a pharmacokinetic data set included in the analysis by evaluating the clinical trial data included.

**6.** The method according to claim 5, wherein the clinical trial data included comprise plasma drug concentration data,

baseline demographic characteristic data, blood biochemical index data and blood collection sites.

**7.** The method according to claim 6, wherein the baseline demographic characteristic data comprise any combination of two or more of race, age, height, weight and gender; and the blood biochemical index data comprise any combination of two or more of blood total protein content, creatinine clearance, glutamic-oxalacetic transaminase, glutamic-pyruvic transaminase, alkaline phosphatase and total bilirubin.

**8.** The method according to claim 3, wherein step (3) comprises determining and processing one or a combination of two or more of observations below a lower limit of detection, anomalous value data, outliers and missing covariates.

**9.** The method according to claim 8, wherein

determining and processing the observations below the lower limit of detection comprises: determining the lower limit of detection by a detecting instrument, wherein the observations below the lower limit of detection are not used for population pharmacokinetic analysis, and if the proportion of the observations below the lower limit of detection is greater than 15%, investigating the influence of the observations below the lower limit of detection on model goodness-of-fit and modeling parameters by using a likelihood function method;
determining and processing the anomalous value data comprises: checking whether there are anomalous values in a sample according to a plasma concentration - time curve in subjects receiving drug administration, and eliminating the anomalous values;
determining and processing the outliers comprises: determining outliers according to residual analysis of preliminary modeling results, and eliminating the outliers; and
processing the missing covariates comprises: if the missing covariate for the subject is less than 15%, applying imputation with the median of the data set for continuous covariates, and applying imputation with a value of the most common category for categorical covariates; and if the missing covariate for the subject is greater than 15%, applying no imputation, and performing exploratory analysis on the PK parameters of the subject with full covariate information by using a Bayesian estimation method.

**10.** The method according to claim 9, wherein the anomalous value data comprise:

1) repeated concentration records at the same time point, except for concentrations in the artery and the vein at the same time point;
2) a trough concentration greater than a corresponding peak concentration;
3) an administration time point after the peak concentration;
4) an administration time point before the trough concentration;
5) concentration records after the end of intravenous administration and before the peak concentration; and
6) unexplained sudden drop or sudden rise in concentrations.

**11.** The method according to claim 3, wherein step (4) comprises comparing various structural models on the basis of a plasma drug concentration-time curve, wherein the optimal model is selected as a preliminary structural model, to form a preliminary foundation model with a residual model.

**12.** The method according to claim 11, wherein the preliminary structural model is a three-compartment model with zero-order absorption and first-order linear elimination from the central compartment, and parameters of the preliminary structural model comprise: central compartment clearance, CL; volume of distribution in the central compartment, V1; volume of distribution in the peripheral compartment 1, V2; volume of distribution in the peripheral compartment 2, V3; intercompartmental clearance between the peripheral compartment 1 and the central compartment, Q2; intercompartmental clearance between the peripheral compartment 2 and the central compartment, Q3; infusion rate, R0; and elimination rate constant K.

**13.** The method according to claim 3, wherein with the preliminary foundation model, interindividual variation of PK parameters is described by using the following equation:

$$\theta_i = \exp(\theta_T + \eta_i)$$

wherein $\theta i$ represents a PK parameter of the ith subject; $\theta_T$ represents a natural logarithm of a population typical value of the PK parameter; and $\eta_i$ represents interindividual variation, and is a random variable following a normal

distribution with mean 0 and variance $\omega^2$, wherein the $\omega^2$ represents a diagonal element of a variance-covariance matrix of the interindividual variation.

**14.** The method according to claim 3, wherein with the preliminary foundation model, variability of a residual is described by using the following equation:

$$log\left(y_{ij}\right)=log\left(\hat{y}_{ij}\right)+\varepsilon_{ij}$$

wherein $y_{ij}$ represents the jth observed concentration of the ith subject, $\hat{y}_{ij}$ represents the jth model-predicted concentration of the ith subject, and $\varepsilon_{ij}$ represents a proportional residual of the jth observed concentration of the ith subject, wherein the observed concentration and the predicted concentration are independent of each other and follow a normal distribution with mean 0 and variance $\sigma$ 2, respectively.

**15.** The method according to claim 3, wherein step (5) comprises determining covariates included in the evaluation on the basis of clinical knowledge and a drug action mechanism, and establishing a final population pharmacokinetic foundation model on the basis of the covariates included in the evaluation.

**16.** The method according to claim 15, wherein the covariates included in the evaluation comprise baseline demographic characteristic covariates, blood biochemical index covariates and blood collection sites.

**17.** The method according to claim 16, wherein the baseline demographic characteristic covariates comprise any combination of two or more of baseline values for age, gender, weight and race; and the blood biochemical index covariates comprise any combination of two or more of creatinine clearance, total protein, glutamic-oxalacetic transaminase, glutamic-pyruvic transaminase, alkaline phosphatase and total bilirubin.

**18.** The method according to claim 3, wherein step (6) comprises

a) pre-screening of covariates; and
b) final screening of the covariates by using a forward method and a backward method, and establishing the population pharmacokinetic model.

**19.** The method according to claim 18, wherein the pre-screening of the covariates comprises:
analyzing the correlation between the PK parameters and each covariate by a graphic method, using linear regression for continuous covariates and using an analysis of variance test for categorical covariates; evaluating the data set on the basis of the model; estimating the parameters of the subject with the final foundation model by using a Bayesian estimation method; and estimating the influence of the covariates on the PK parameters.

**20.** The method according to claim 19, wherein the pre-screening of the covariates comprises:
analyzing the correlation between the continuous covariates and the PK parameters by using the following equation:

$$\theta_i=\theta_{pop}\cdot\left(\frac{Cov_i}{Cov_{pop}}\right)^{k_{\text{cov}}};$$

and analyzing the correlation between the categorical covariates and the PK parameters by using the following equation:

$$\theta_i=\theta_{pop}\times\exp(k_{\text{cov}}\cdot X_i)$$

wherein $\theta_i$ represents a PK parameter of the ith subject; $\theta_{pop}$ represents a test population typical value of the PK parameter; $Cov_i$ represents a continuous covariate of the ith subject; $Cov_{pop}$ represents a median of the continuous variables in a test population; $X_i$ represents a categorical variable index of the ith subject, wherein value 0 represents the category with the most common category of a covariate, while other integer values represent other categories

respectively; and $K_{cov}$ represents a coefficient describing the influence of the covariate.

**21.** The method according to claim 20, wherein the final screening of the covariates comprises:

establishing a full model by using a forward method on the basis of the final foundation model, and then establishing the population pharmacokinetic model by using a backward method on the basis of the full model, wherein the forward method comprises: sequentially adding each covariate to a preliminary structural model of the final foundation model in step (5), wherein with a log likelihood ratio test, if an objective function value is decreased by more than 6.63 after adding 1 covariate, the newly added covariate is considered significant, with $p < 0.01$; and firstly adding the covariate having the most significant influence on the basis of the preliminary structural model to form an improved model, then testing the statistically significant covariate screened in the previous step with the improved model, and repeating the process until no significant covariates can be found; and

the backward method comprises: a process of deleting the covariates one by one on the basis of the full model, wherein if an objective function value is increased by more than 10.83 after deleting 1 covariate, the deleted covariate is considered significant, with $p < 0.001$.

**22.** The method according to claim 3, wherein step (7) comprises evaluating the population pharmacokinetic model by using one or a combination of two or more of the following methods: a model goodness-of-fit diagnostic plot, a visual predictive check, bootstrap and shrinkage.

**23.** The method according to claim 22, wherein

the model goodness-of-fit diagnostic plot comprises one or a combination of two or more of the following figures: a relation diagram of a population predicted concentration and an observed concentration, a relation diagram of an individual predicted concentration and an observed concentration, a relation diagram of a conditional weighted residual and a population predicted concentration, and a relation diagram of a conditional weighted residual and time after first administration;

the visual predictive check comprises comparative mapping of a predicted result and a measured value according to final model parameters, covariates and actual dosage of administration so as to evaluate whether the population pharmacokinetic model can well describe a plasma drug concentration-time curve of a propofol derivative;

the bootstrap comprises repeatedly fitting with population pharmacokinetic model until 1000 data sets for bootstrap replication, and randomly selecting subject data and covariates for replacement to achieve the replication; and

the shrinkage comprises estimating individual parameter values of a subject by using a Bayesian estimation method with the population pharmacokinetic model, and calculating interindividual variations and individual residuals from model predictions and observations.

**24.** The method according to claim 23, wherein the shrinkage comprises evaluating the interindividual variations and individual residuals of the pharmacokinetic parameters by using the following equation, and quantifying the individual parameter values and random error estimates:

$$\eta_{shrinkage} = 1 - \frac{SD\left(\hat{\eta}_{ph}\right)}{\omega}$$

$$\varepsilon_{shrinkage} = 1 - SD\left(IWRES\right);$$

wherein $\eta_{shrinkage}$ is interindividual variation, $\varepsilon_{shrinkage}$ is an individual residual, $\omega$ is interindividual variation degree of individual parameter values estimated by the population pharmacokinetic model, $\eta_{ph}$ is $\eta$ value of the parameter for all individuals, IWRES is an individual weighted residual; and SD represents standard deviation.

**25.** The method according to claim 3, wherein step (7) further comprises estimating individual PK parameters of the subject by using a Bayesian post-hoc method, simulating a plasma drug concentration-time curve of intravenous infusion according to actual dosages of administration, and calculating the area under the plasma drug concentration-time curve from 0-1 min, the area under the plasma drug concentration-time curve from 0-2 min, the area under the

plasma drug concentration-time curve from 0-4 min, the area under the plasma drug concentration-time curve from 0-10 min, the area under the plasma drug concentration-time curve from 0-24 h and the peak concentration.

**26.** A system for determining individual administration parameters of a compound of formula (I) or propofol, the system comprising a data acquisition device, a data processing device and a result output device,

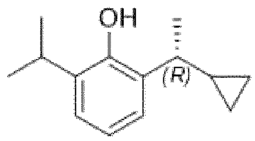 formula (I)

wherein the data comprise baseline demographic characteristic data, blood biochemical index data and blood collection site information data; with the data processing device, individual administration parameter results of the compound of formula (I) are obtained by using the following equation:

$$CL_i = \exp(4.20+0.349 \cdot \log(WT / 63.9) - 0.749 \cdot \log(TP / 72.4) + 0.238 \cdot SITE + \eta_{CL,i});$$

or individual administration parameter results of propofol are obtained by using the following equation:

$$CL_i = \exp(4.56 + \eta_{CL,i})$$

wherein CLi represents central compartment clearance of the ith subject; when collecting a sample from venous blood, SITE = 0, and when collecting a sample from artery blood, SITE = 1; WT represents weight; TP represents total protein; $\eta_{CL,i}$ is interindividual variation of the CL of the ith subject; and $\eta$ follows a normal distribution with mean 0 and variance $\omega$2, wherein the $\omega$2 is a diagonal element of a variance-covariance matrix S2 of the interindividual variation.

**27.** The system according to claim 26, wherein with the data processing device, individual administration parameter results of the compound of formula (I) are obtained by using the following equation:

$$CL_i = \exp(4.20+0.349 \cdot \log(WT / 63.9) - 0.749 \cdot \log(TP / 72.4) + 0.238 \cdot SITE + \eta_{CL,i})$$

$$V_{1i} = \exp(0.908 + 0.426 \cdot \log(AGE / 27) + \eta_{V_1,i})$$

$$Q_{2i} = \exp(4.06 + \eta_{Q_2,i})$$

$$V_{2i} = \exp(1.75)$$

$$Q_{3i} = \exp(4.08 + \eta_{Q_3,i})$$

$$V_{3i} = \exp(4.36 + \eta_{V_3,i})$$

or individual administration parameter results of propofol are obtained by using the following equation:

$$CL_i = \exp(4.56 + \eta_{CL,i})$$

$$V_{1i} = \exp(2.25 + \eta_{V_1,i})$$

$$Q_{2i} = \exp(4.96)$$

$$V_{2i} = \exp(3.63)$$

$$Q_{3i} = \exp(3.88)$$

$$V_{3i} = \exp(5.57)$$

wherein $V_{1i}$ represents volume of distribution in the central compartment of the ith subject;
$V_{2i}$ represents volume of distribution in the peripheral compartment 1 of the ith subject;
$V_{3i}$ represents volume of distribution in the peripheral compartment 2 of the ith subject;
$Q_{2i}$ represents intercompartmental clearance between the peripheral compartment 1 and the central compartment of the ith subject;
$Q_{3i}$ represents intercompartmental clearance between the peripheral compartment 2 and the central compartment of the ith subject;
AGE represents age; and $\eta$ represents interindividual variation of a corresponding parameter.

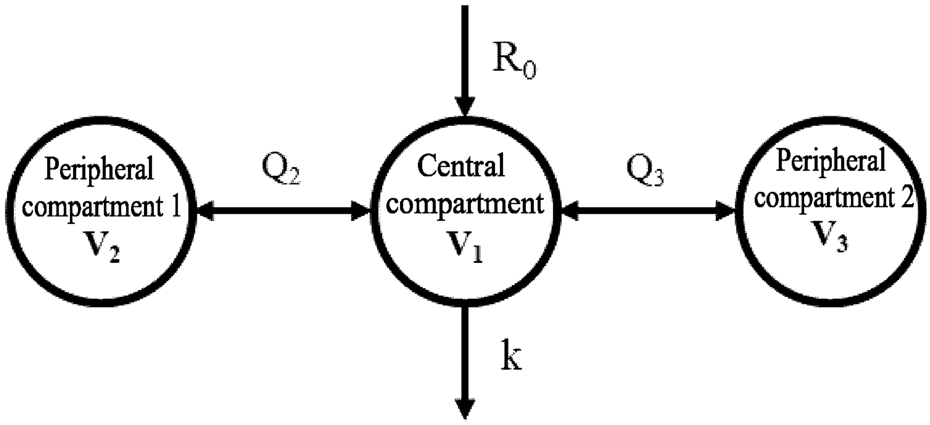
R0
Peripheral compartment 1
V2
Q2
Central compartment
V1
Q3
Peripheral compartment 2
V3
k

*FIG. 1*

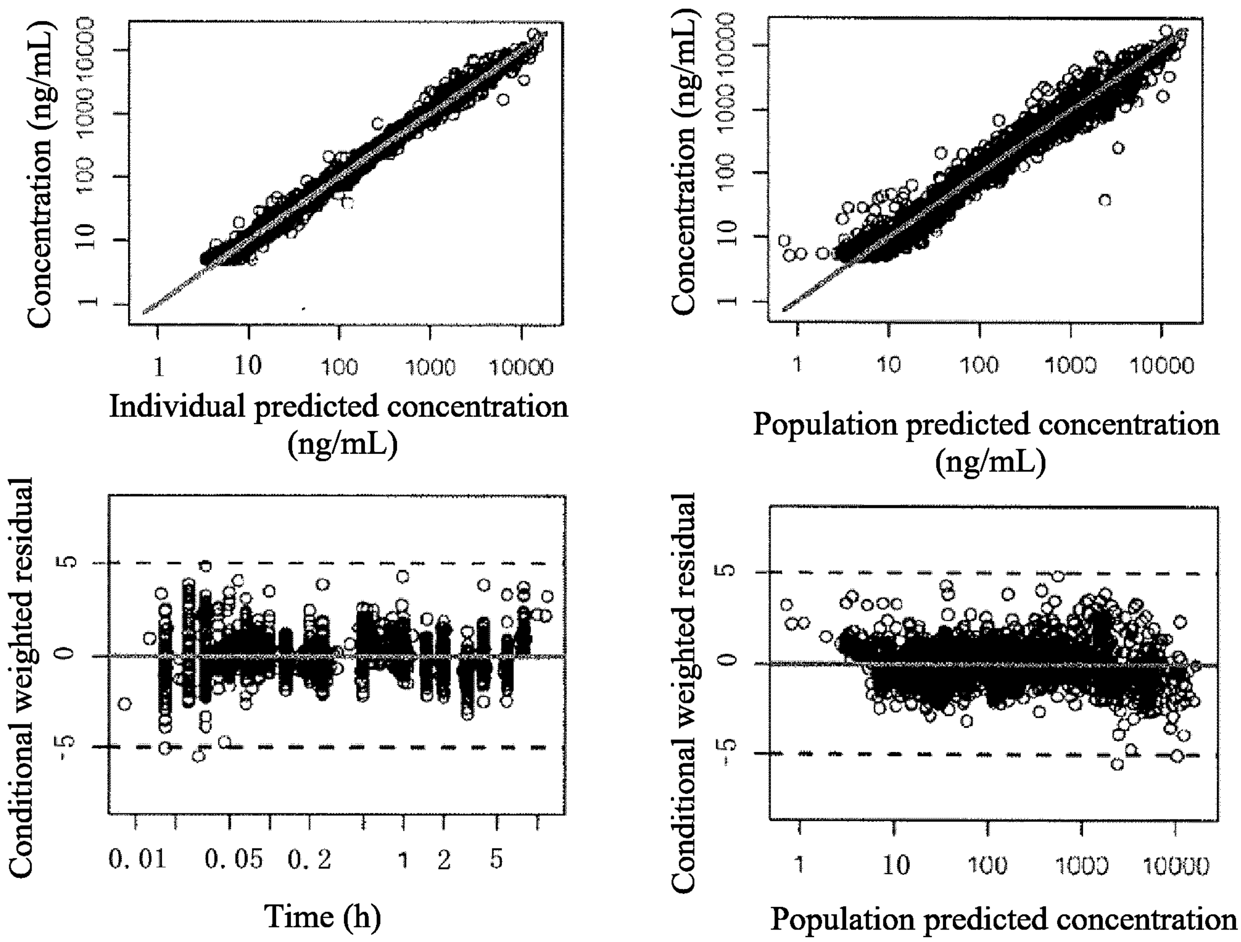
Concentration (ng/mL)
1
10
100
1000
10000
Individual predicted concentration (ng/mL)
Population predicted concentration (ng/mL)
Conditional weighted residual
5
0
-5
0.01
0.05
0.2
1
2
5
Time (h)
Population predicted concentration

*FIG. 2*

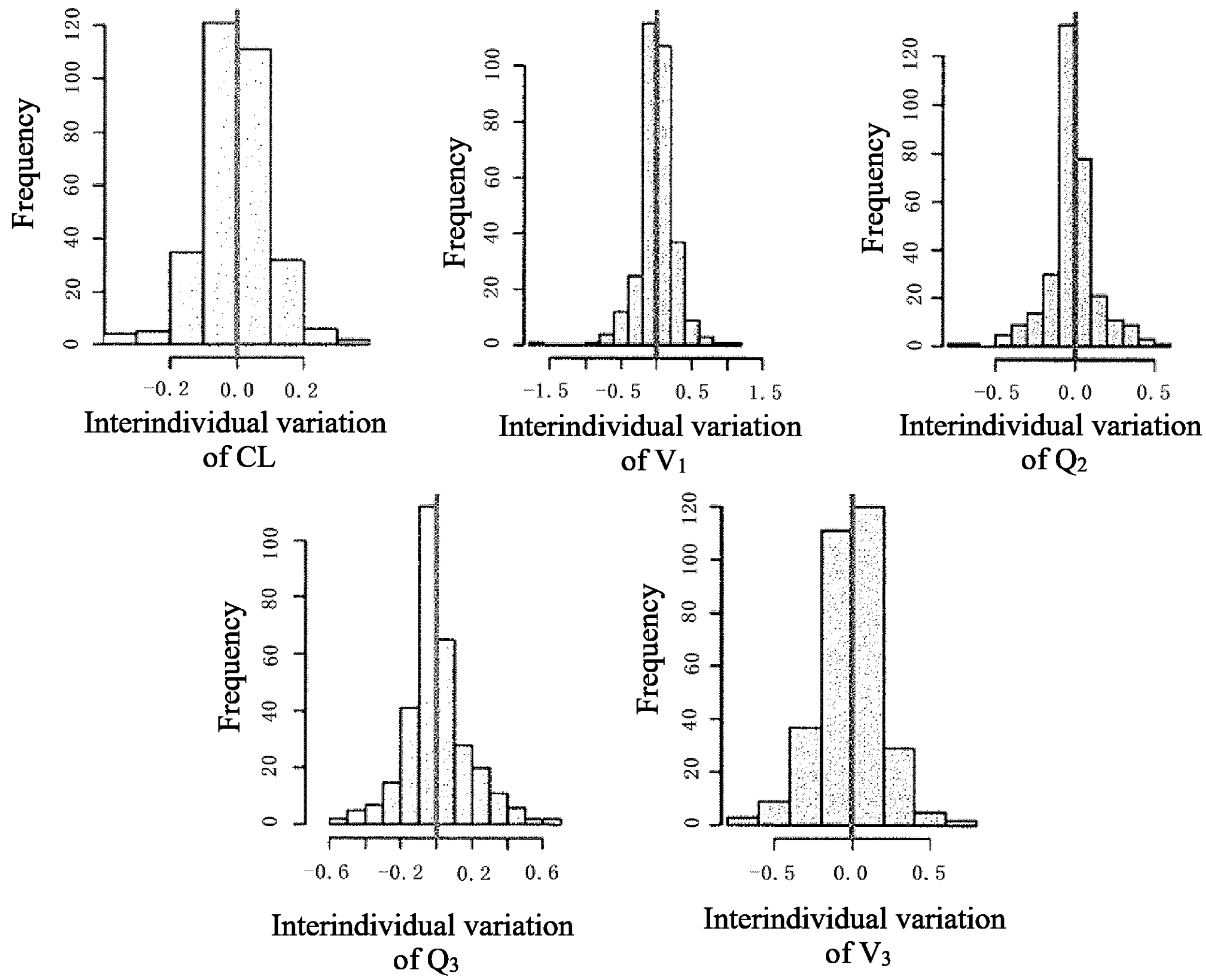
Frequency
Interindividual variation of CL
Interindividual variation of V1
Interindividual variation of Q2
Interindividual variation of Q3
Interindividual variation of V3

*FIG. 3*

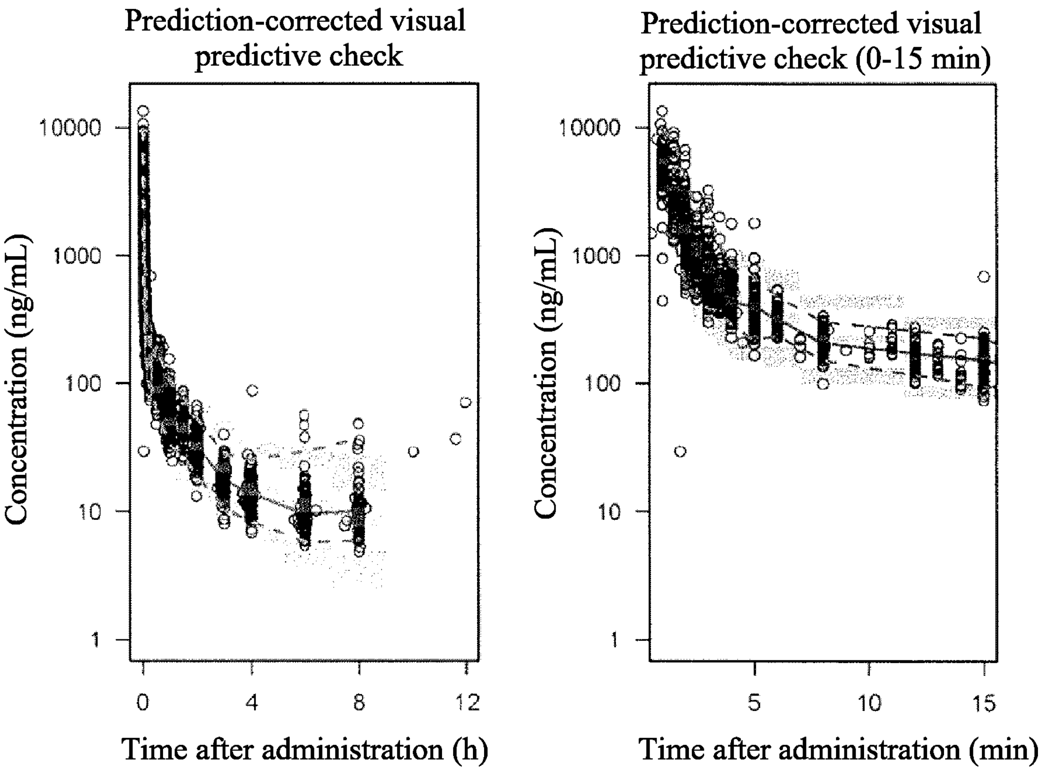
Prediction-corrected visual predictive check
Prediction-corrected visual predictive check (0-15 min)
Concentration (ng/mL)
10000
1000
100
10
1
0
4
8
12
5
10
15
Time after administration (h)
Time after administration (min)

*FIG. 4*

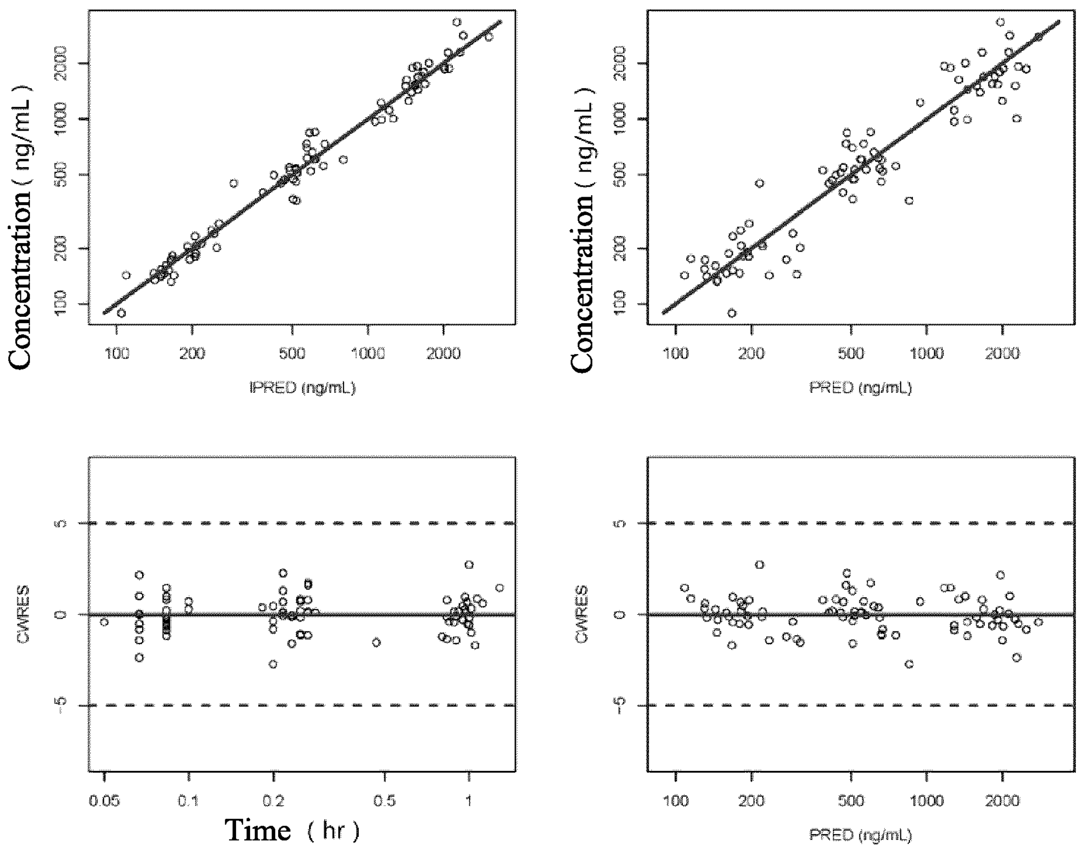
Concentration ( ng/mL )
100
200
500
1000
2000
IPRED (ng/mL)
PRED (ng/mL)
CWRES
5
0
-5
0.05
0.1
0.2
0.5
1
Time ( hr )

*FIG. 5*

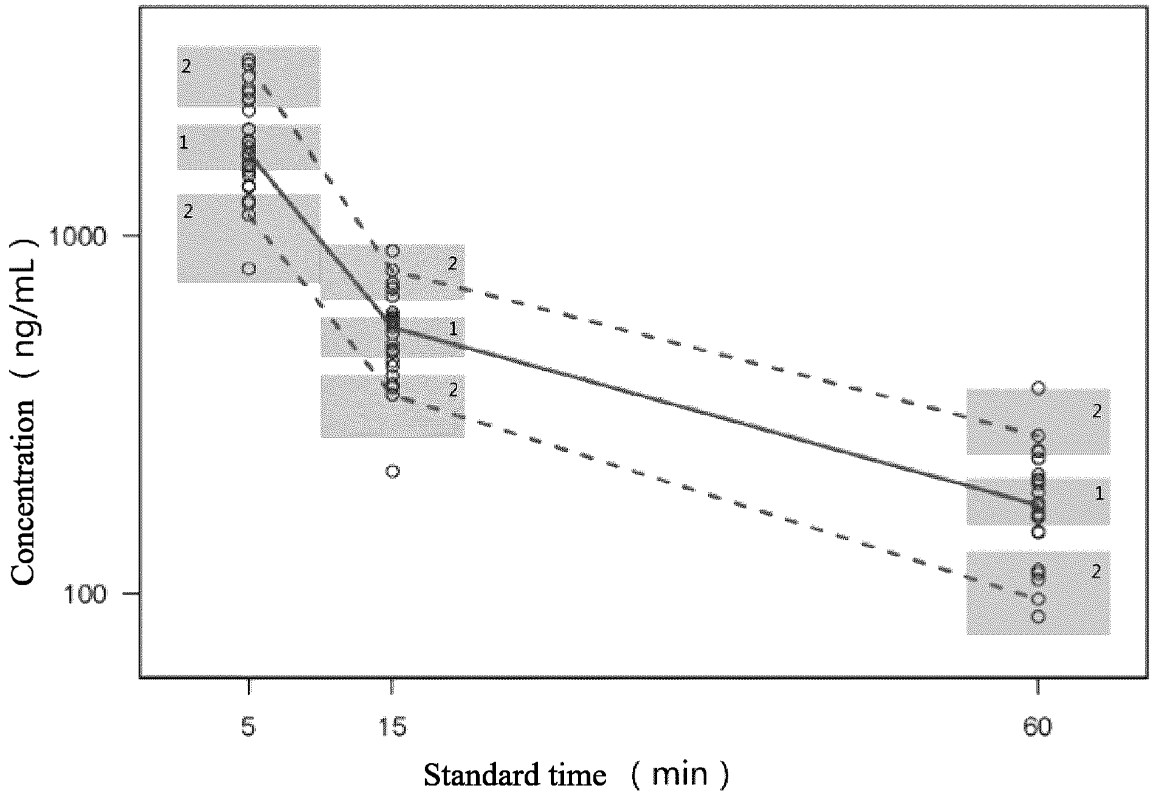
1000
100
Concentration（ng/mL）
2
1
2
2
1
2
2
1
2
5
15
60
Standard time （min）

*FIG. 6*

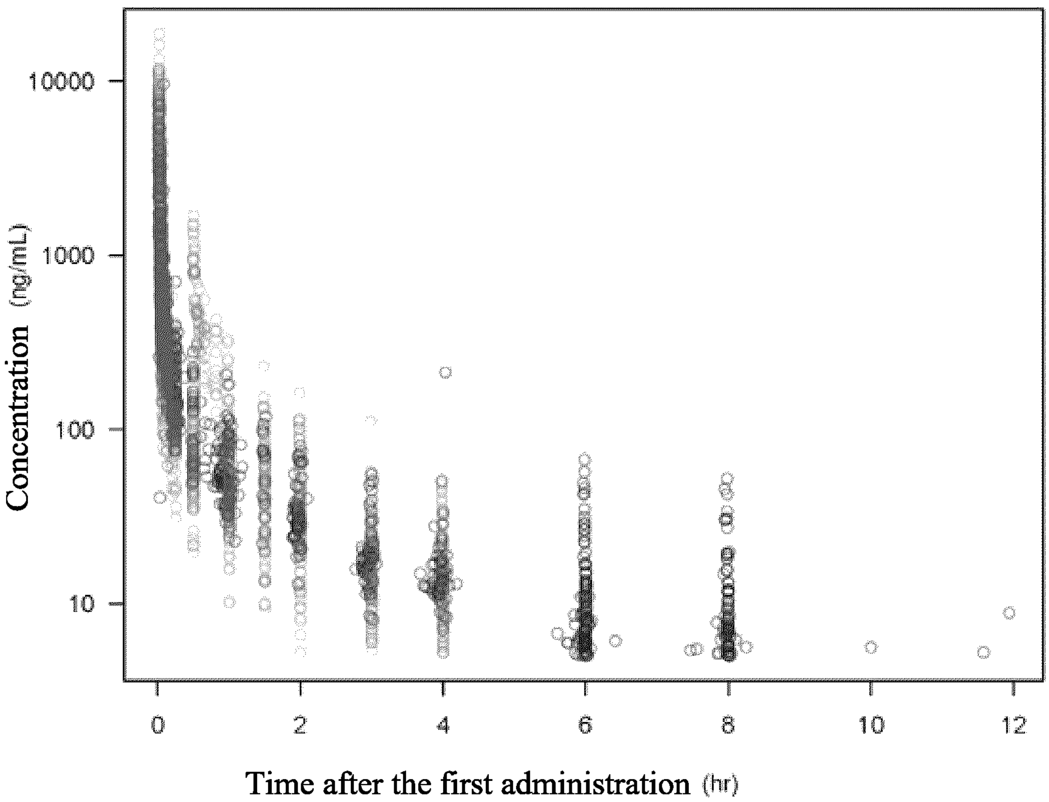
10000
1000
100
10
Concentration (ng/mL)
0
2
4
6
8
10
12
Time after the first administration (hr)

*FIG. 7*

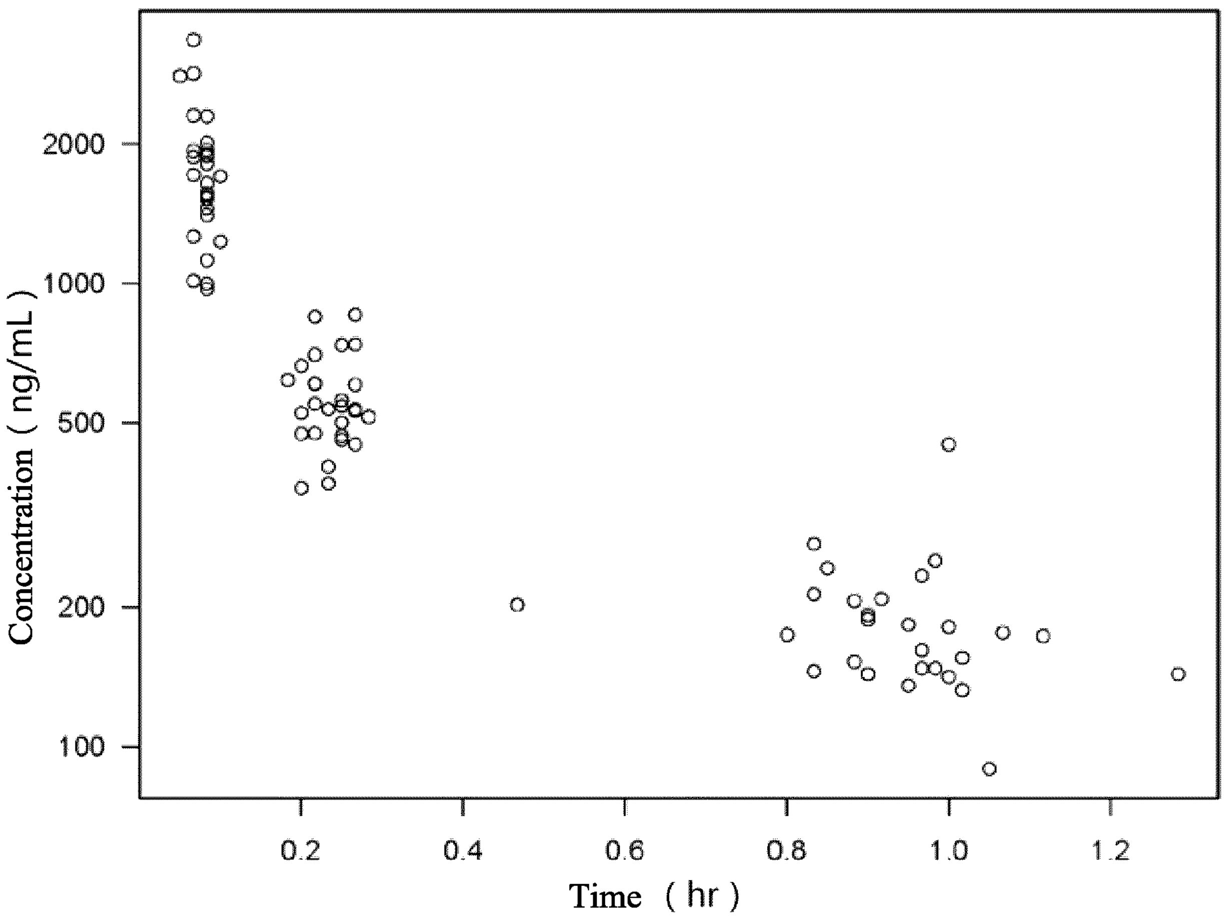

2000
1000
500
200
100
Concentration ( ng/mL )
0.2
0.4
0.6
0.8
1.0
1.2
Time ( hr )


*FIG. 8*

**INTERNATIONAL SEARCH REPORT**

International application No. **PCT/CN2021/109601**

**A. CLASSIFICATION OF SUBJECT MATTER**

G16B 5/00(2019.01)i； A61B 5/00(2006.01)i； A61P 23/00(2006.01)i

According to International Patent Classification (IPC) or to both national classification and IPC

**B. FIELDS SEARCHED**

Minimum documentation searched (classification system followed by classification symbols)

G16B5/-; A61B5/-; A61P23/-

Documentation searched other than minimum documentation to the extent that such documents are included in the fields searched

Electronic data base consulted during the international search (name of data base and, where practicable, search terms used)

WPI, EPODOC, CNPAT, CNKI, Caplus(STN), Registry(STN), 丙泊酚, 药代动力学模型, propofol, pharmacokinetic model

**C. DOCUMENTS CONSIDERED TO BE RELEVANT**

| Category* | Citation of document, with indication, where appropriate, of the relevant passages | Relevant to claim No. |
|---|---|---|
| A | ABSALOM A.R.et al. "Pharmacokinetic models for propofol—defining and illuminating the devil in the detail" *British Journal of Anaesthesia,* Vol. 103, No. 1, 10 June 2009 (2009-06-10), pp. 26-37 | 1-27 |
| A | CN 109069736 A (FRESENIUS VIAL SAS) 21 December 2018 (2018-12-21) claims 4-10 | 1-27 |
| A | CN 110269590 A (SHANGHAI UNITED IMAGING HEALTHCARE CO., LTD.) 24 September 2019 (2019-09-24) description, paragraphs [0062]-[0105] | 1-27 |
| A | CN 110251770 A (THE SECOND AFFILIATED HOSPITAL & YUYING CHILDREN'S HOSPITAL OF WENZHOU MEDICAL UNIVERSITY et al.) 20 September 2019 (2019-09-20) description, paragraphs [0002]-[0006] | 1-27 |
| A | CN 105844112 A (BEIJING DRYAS PHARMA-TECH CO., LTD.) 10 August 2016 (2016-08-10) embodiments 1-2 | 1-27 |
| A | WO 0223186 A2 (VIRCO N.V. et al.) 21 March 2002 (2002-03-21) claims 1-45 | 1-27 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 October 2021** | **01 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/109601**

| Patent document cited in search report | | | Publication date (day/month/year) | Patent family member(s) | | | Publication date (day/month/year) |
|---|---|---|---|---|---|---|---|
| CN | 109069736 | A | 21 December 2018 | IL | 262535 | D0 | 31 December 2018 |
| | | | | US | 2019143040 | A1 | 16 May 2019 |
| | | | | RU | 2713972 | C1 | 11 February 2020 |
| | | | | JP | 2019523659 | A | 29 August 2019 |
| | | | | BR | 112018071795 | A2 | 19 February 2019 |
| | | | | WO | 2017190966 | A1 | 09 November 2017 |
| | | | | EP | 3452145 | A1 | 13 March 2019 |
| CN | 110269590 | A | 24 September 2019 | | None | | |
| CN | 110251770 | A | 20 September 2019 | CN | 210963374 | U | 10 July 2020 |
| CN | 105844112 | A | 10 August 2016 | | None | | |
| WO | 0223186 | A2 | 21 March 2002 | AU | 2002212273 | B2 | 14 June 2007 |
| | | | | US | 2004023211 | A1 | 05 February 2004 |
| | | | | EP | 1328806 | A2 | 23 July 2003 |
| | | | | JP | 2004510961 | A | 08 April 2004 |
| | | | | CA | 2419244 | A1 | 21 March 2002 |
| | | | | WO | 0223186 | A3 | 22 August 2002 |
| | | | | US | 2008008991 | A1 | 10 January 2008 |
| | | | | AU | 1227302 | A | 26 March 2002 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016034079 A